(19) Europäisches Patentamt
European Patent Office
Office européen des brevets

(11) EP 4 659 651 A2

(12) **EUROPEAN PATENT APPLICATION**

(43) Date of publication:
**10.12.2025 Bulletin 2025/50**

(21) Application number: **25204354.2**

(22) Date of filing: **24.09.2025**

(51) International Patent Classification (IPC):
***A61B 1/00*** *(2006.01)* ***A61B 1/05*** *(2006.01)*

(52) Cooperative Patent Classification (CPC):
**A61B 1/00096; A61B 1/00188; A61B 1/05**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **25.09.2024 CN 202411346449**

(71) Applicant: **Jiangxi OFILM Optical Co., Ltd.**
**Nanchang, Jiangxi 330096 (CN)**

(72) Inventors:
- **ZHANG, Chenglin**
  **Nanchang, 330096 (CN)**
- **YIN, Jia**
  **Nanchang, 330096 (CN)**
- **WANG, Guogui**
  **Nanchang, 330096 (CN)**
- **LIU, Binbin**
  **Shenzhen, 518106 (CN)**

(74) Representative: **Metida**
**Gyneju str. 16**
**01109 Vilnius (LT)**

(54) **OPTICAL LENS, IMAGE MODULE, AND ENDOSCOPE**

(57) An optical lens (100) includes a first lens (L1) having negative refractive power, a second lens (L2) having positive power, a third lens (L3) having positive refractive power, a fourth lens (L4) having negative refractive power, and an image plane (IMG). An imaging side surface (S2) of the first lens (L1) is concave near the optical axis (O), object side surfaces (S3, S5) of the second lens (L2) and the third lens (L3) are convex near the optical axis (O), and an object side surface (S7) of the fourth lens (L4) is concave near the optical axis (O). The optical lens (100) satisfies: 110deg<FOV<155deg, and 1.7<TTL/ImgH<2.7.

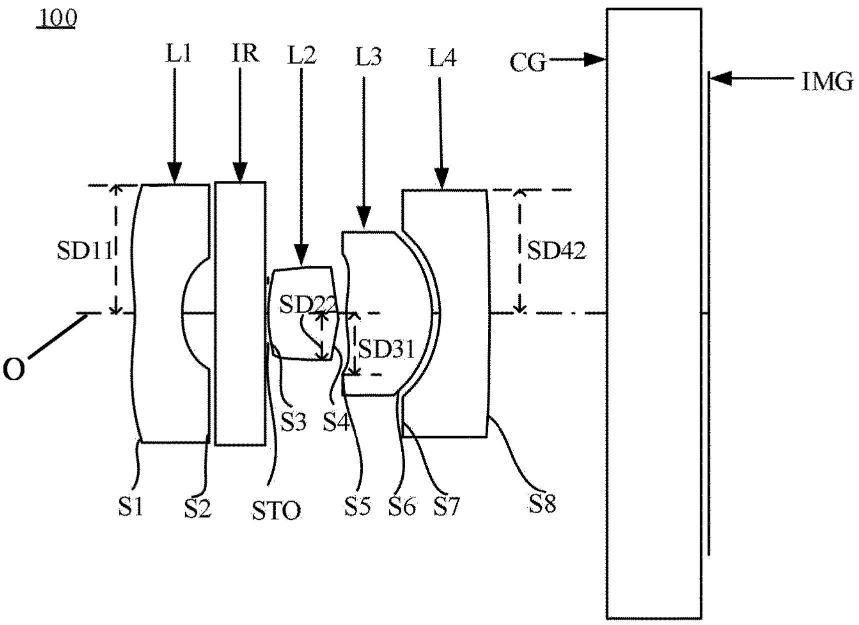


FIG.1A

EP 4 659 651 A2

**Description**

FIELD

The present invention relates to field of imaging, and in particular to an optical lens, an image module, and an endoscope.

BACKGROUND

Medical endoscopes are mainly auxiliary medical devices that improve diagnostic accuracy by inserting tubes equipped with camera modules into the human body and capturing internal images. In related technologies, considering that endoscopes usually need to be inserted into the human body, to minimize harm to patients, it is typically required that endoscopes have good insertability, that is, the outer diameter of the head end should be small, the bending hard end should be short, and the observation range should be large. In other words, endoscopes should have the characteristics of compact structure and large field of view. Therefore, how to balance the compactness and large field of view of endoscopes is an urgent problem to be solved.

SUMMARY

The present disclosure discloses an optical lens, an image module, and an endoscope, which may effectively balance a compact structure of the endoscope and the requirement of a large field of view angle.

In order to achieve the above objects, in a first aspect, the present application discloses an optical lens. The optical lens sequentially includes a first lens, a filter, a second lens, a third lens, a fourth lens, and an image plane along an optical axis from an object side to an imaging side. The first lens has negative refractive power. An imaging side surface of the first lens is concave near the optical axis. The second lens has positive refractive power, and an object side surface of the second lens is convex near the optical axis. The third lens has positive refractive power, an object side surface of the third lens is convex near the optical axis. The fourth lens has negative refractive power, and an object side surface of the fourth lens is concave near the optical axis. The optical lens satisfying following conditional expressions: $110deg<FOV<155deg$, and $1.7<TTL/ImgH<2.7$. FOV is the maximum field of view angle of the optical lens, TTL is a distance from an object side surface of the first lens to the image plane of the optical lens along the optical axis, and ImgH is half of an image height corresponding to the maximum field of view of the optical lens.

In some embodiments, the optical lens further satisfies at least one of following conditional expressions: $-1.4<f1/f<-0.6$, $1.2<f2/f<4$, $0.5<f3/f<1.1$, and $-2<f4/f<-0.8$. Wherein, f1 is a focal length of the first lens, f is a focal length of the optical lens, f2 is a focal length of the second lens, f3 is a focal length of the third lens, and f4 is a focal length of the fourth lens.

In some embodiments, the optical lens further satisfies at least one of following conditional expressions: $0.7<|R11|/f$ and$0.3<R12/f$. Wherein, R11 is a radius of curvature of an object side surface of the first lens at the optical axis, f is a focal length of the optical lens, and R12 is a radius of curvature of the imaging side surface of the first lens at the optical axis.

In some embodiments, the optical lens further satisfies at least one of following conditional expressions: $0.6<R21/f<2$ and$9<|R22|/f$. Wherein, R21 is a radius of curvature of the object side surface of the second lens at the optical axis, f is a focal length of the optical lens, and R22 is a radius of curvature of an imaging side surface of the second lens at the optical axis.

In some embodiments, the optical lens further satisfies at least one of following conditional expressions: $0.5<R31/f <1.0$ and $-1.1<R32/<-0.4$. Wherein, R31 is a radius of curvature of the object side surface of the third lens at the optical axis, f is a focal length of the optical lens, and R32 is a radius of curvature of an imaging side surface of the third lens at the optical axis.

In some embodiments, the optical lens further satisfies at least one of following conditional expressions: $-1.3<R41/f<-0.4$ and $3<|R42|/f$. Wherein, R41 is a curvature radius of the object side surface of the fourth lens at the optical axis, f is a focal length of the optical lens, and R42 is a curvature radius of an imaging side surface of the fourth lens at the optical axis.

In some embodiments, the optical lens further satisfies at least one of following conditional expressions: $2.5<TTL/f<5.5$ and $1.2<ImgH/f<2.3$. Wherein, f is a focal length of the optical lens.

In some embodiments, the optical lens further satisfies at least one of following conditional expressions: $2.2<TTL/BL<3$ and $2.5<TD/\Sigma AT<3.5$. Wherein, BL is a distance from an imaging side surface of the fourth lens to the image plane of the optical lens along the optical axis, TD is a distance from an object side surface of the first lens to the imaging side surface of the fourth lens at the optical axis, and $\Sigma AT$ is a sum of distances from the imaging side surface of the first lens to an object side surface of the filter, from an imaging side surface of the filter to the object side surface of the second lens, from an imaging side surface of the second lens to the object side surface of the third lens, and from an imaging side surface of the third lens to the object side surface of the fourth lens at the optical axis.

In some embodiments, the optical lens further satisfies at least one of following conditional expressions: $3<FNO<5$ and $26deg<FOV/FNO<40deg$. Wherein, FNO is an aperture number of the optical lens.

In some embodiments, the optical lens further satisfies at least one of following conditional expressions: $0.9<CT3/CT4<2.1$, $1<ET1/CT1<1.9$, and$1<ET4/CT4<2.1$. Wherein, CT3 is a thickness of the third lens at the optical axis, CT4 is a thickness of the fourth lens at the optical axis, ET1 is a distance from the maximum effective semi-aperture of an object side surface of the first lens to the maximum effective semi-aperture of the imaging side surface of the first lens, and ET4 is a distance from the maximum effective semi-aperture of the object side surface of the fourth lens to the maximum effective semi-aperture of an imaging side surface of the fourth lens.

In some embodiments, the optical lens further satisfies following conditional expression: $6<AT12/AT34<22$. Wherein, AT12 is a sum of distances from the imaging side surface of the first lens to an object side surface of the filter and from an imaging side surface of the filter to the object side surface of the second lens at the optical axis, and AT34 is a distance from an imaging side surface of the third lens to the object side surface of the fourth lens at the optical axis.

In some embodiments, the optical lens further satisfies at least one of following conditional expressions: $1<SD11/SD42<1.3$, and $0.8<SD22/SD31<1$. Wherein, SD11 is the maximum effective semi-aperture of an object side surface of the first lens, SD42 is the maximum effective semi-aperture of an imaging side surface of the fourth lens, SD22 is the maximum effective semi-aperture of an imaging side surface of the second lens, and SD31 is the maximum effective semi-aperture of the object side surface of the third lens.

In some embodiments, the optical lens further satisfies following conditional expression: $0.4<SD11/ImgH<0.6$. Wherein, SD11 is the maximum effective semi-aperture of an object side surface of the first lens.

In some embodiments, the optical lens further satisfies at least one of following conditional expressions: $0.5<(|SAG11|+|SAG12|)/CT1<1.5$, $0.1<(|SAG21|+|SAG22|)/CT2<0.4$, $0.1<(|SAG31|+|SAG32|)/CT3<0.9$, and$0.3<(|SAG41|+|SAG42|)/CT4<1$. Wherein, CT1 is a thickness of the first lens at the optical axis, SAG11 is a distance from the maximum effective semi-aperture of an object side surface of the first lens to an intersection point of the object side surface of the first lens and the optical axis in the direction of the optical axis, SAG12 is a distance from the maximum effective semi-aperture of the imaging side surface of the first lens to an intersection point of the imaging side surface of the first lens and the optical axis in the direction of the optical axis, CT2 is a thickness of the second lens at the optical axis, SAG21 is a distance from the maximum effective semi-aperture of the object side surface of the second lens to an intersection point of the object side surface of the second lens and the optical axis in the direction of the optical axis, SAG22 is a distance from the maximum effective semi-aperture of an imaging side surface of the second lens to an intersection point of the imaging side surface of the second lens and the optical axis in the direction of the optical axis, CT3 is a thickness of the third lens at the optical axis, SAG31 is a distance from the maximum effective semi-aperture of the object side surface of the third lens to an intersection point of the object side surface of the third lens and the optical axis in the direction of the optical axis, SAG32 is a distance from the maximum effective semi-aperture of an imaging side surface of the third lens to an intersection point of the imaging side surface of the third lens and the optical axis in the direction of the optical axis, CT4 is a thickness of the fourth lens at the optical axis, SAG41 is a distance from the maximum effective semi-aperture of the object side surface of the fourth lens to an intersection point of the object side surface of the fourth lens and the optical axis in the direction of the optical axis, and SAG42 is a distance from the maximum effective semi-aperture of an imaging side surface of the fourth lens to an intersection point of the imaging side surface of the fourth lens and the optical axis in the direction of the optical axis.

In some embodiments, the optical lens further satisfies at least one of following conditional expressions: $-14<f4/|SAGY41|<-3$ and $f4/|SAGY42|<-10$. Wherein, SAG41 is a distance from the maximum effective semi-aperture of the object side surface of the fourth lens to an intersection point of the object side surface of the fourth lens and the optical axis in the direction of the optical axis, SAG42 is a distance from the maximum effective semi-aperture of an imaging side surface of the fourth lens to an intersection point of the imaging side surface of the fourth lens and the optical axis in the direction of the optical axis, and f4 is a focal length of the fourth lens.

In some embodiments, the optical lens further satisfies at least one of following conditional expressions: $2.3<R11/R12<100$, $7<|R22/R21|<30$, $-1.7<R31/R32<-0.6$, and $4<|R42/R41|$. Wherein, R11 is a radius of curvature of an object side surface of the first lens at the optical axis, R12 is a radius of curvature of the imaging side surface of the first lens at the optical axis, R21 is a radius of curvature of the object side surface of the second lens at the optical axis, R22 is a radius of curvature of an imaging side surface of the second lens at the optical axis, R31 is a radius of curvature of the object side surface of the third lens at the optical axis, R32 is a radius of curvature of an imaging side surface of the third lens at the optical axis, R41 is a curvature radius of the object side surface of the fourth lens at the optical axis, and R42 is a curvature radius of an imaging side surface of the fourth lens at the optical axis.

In some embodiments, the optical lens further comprises an aperture, and the aperture is arranged between the filter and the object side surface of the second lens.

In some embodiments, the optical lens further comprises a protective glass, and the protective glass is arranged between an imaging side surface of the fourth lens and the image plane of the optical lens.

In a second aspect, the present application discloses an image module. The image module includes an imaging sensor and the above-mentioned optical lens, the imaging sensor is arranged on the imaging side of the optical lens. The image module with the above optical lens can achieve a large field of view while taking into account the miniaturization design.

In a third aspect, the present application discloses an endoscope. The endoscope includes a tube body and the above-mentioned image module, the image module is arranged in the tube body. The endoscope with the above-mentioned image module can achieve a large field of view while taking into account the miniaturization design.

Compared with the existing technology, the beneficial effects of the present application lie in that: in the optical lens provided by the present application, in order to achieve a large field of view on the basis of miniaturization design, the present application sets up four refractive lenses and places the filter between the first lens and the second lens, thereby enabling the aperture to change from large to small. On this basis, the present application also designs the refractive power and surface shape of the four lenses. Specifically, the first lens has negative refractive power, and its image side surface is concave near the optical axis, which is conducive to the convergence of light rays in a large field of view range and can also make the head diameter of the optical lens smaller. The second lens has positive refractive power, and its object side surface is convex near the optical axis, which can effectively correct the aberration produced by the first lens and improve the imaging quality of the optical lens. The third lens has positive refractive power, and its object side surface is convex near the optical axis, which can help reduce the incident angle of the light rays entering the optical lens, allowing as many light rays as possible to enter the optical lens. The fourth lens has negative refractive power, and its object side surface is concave near the optical axis, which can help correct the spherical aberration, coma aberration and distortion produced by the first, second and third lenses, further improving the imaging quality of the optical lens.

In addition, when the optical lens satisfies the conditional expressions $110deg<FOV<155deg$ and $1.7<TTL/ImgH<2.7$, it can achieve a large field of view imaging effect while realizing miniaturization design, which is conducive to improving the imaging quality of the optical lens.

## BRIEF DESCRIPTION OF THE DRAWINGS

To better describe and illustrate embodiments and/or examples of those inventions disclosed herein, reference may be made to one or more of the accompanying drawings. The additional details or examples used to describe the drawings should not be construed as limiting the scope of any of the disclosed inventions, the embodiments and/or examples presently described, and the best modes currently understood of these inventions.

FIG. 1A is a schematic structure diagram of an optical lens of a first embodiment of the present application.
FIG. 1B is a schematic structure diagram of lenses of the first embodiment of the present application.
FIG. 2 is a longitudinal spherical aberration diagram (mm), astigmatism curve diagram (mm) and distortion diagram (%) of the optical lens of the first embodiment of the present application.
FIG. 3 is a schematic structure diagram of an optical lens of a second embodiment of the present application.
FIG. 4 is a longitudinal spherical aberration diagram (mm), astigmatism curve diagram (mm) and distortion diagram (%) of the optical lens of the second embodiment of the present application.
FIG. 5 is a schematic structure diagram of an optical lens of a third embodiment of the present application.
FIG. 6 is a longitudinal spherical aberration diagram (mm), astigmatism curve diagram (mm) and distortion diagram (%) of the optical lens of the third embodiment of the present application.
FIG. 7 is a schematic structure diagram of an optical lens of a fourth embodiment of the present application.
FIG. 8 is a longitudinal spherical aberration diagram (mm), astigmatism curve diagram (mm) and distortion diagram (%) of the optical lens of the fourth embodiment of the present application.
FIG. 9 is a schematic structure diagram of an optical lens of a fifth embodiment of the present application.
FIG. 10 is a longitudinal spherical aberration diagram (mm), astigmatism curve diagram (mm) and distortion diagram (%) of the optical lens of the fifth embodiment of the present application.
FIG. 11 is a schematic diagram of an image module of an embodiment of the present application.
FIG. 12 is a schematic diagram of an endoscope of an embodiment of the present application.

## DETAILED DESCRIPTION

The following will describe the technical solutions of the embodiments of the present disclosure clearly and completely in combination with the accompanying drawings of the embodiments of the present disclosure. Obviously, the described embodiments are only part of the embodiments of the present disclosure, not all of them. Based on the embodiments of the present disclosure, those skilled in the art can make various modifications or variations without departing from the spirit or scope of the present disclosure. All other embodiments obtained by persons of ordinary skill in the art without making creative efforts fall within the scope of protection of the present disclosure.

In addition, the terms "first", "second", etc. are mainly used to distinguish different devices, components, or parts (the specific types and constructions may be the same or different), and are not intended to indicate or imply the relative importance and quantity of the indicated devices, components, or parts. Unless otherwise specified, "multiple" means two or more.

The technical solution of the present application will be further explained in conjunction with the embodiments and accompanying drawings.

Referring to FIG. 1A and FIG. 1B, in some embodiments of the present application, an optical lens 100 has a total of four refractive lenses. Along an optical axis O from an object side to an imaging side, the optical lens 100 sequentially includes a first lens L1, a filter IR, a second lens L2, a third lens L3, and a fourth lens L4.

In some embodiments, the first lens L1 may have negative refractive power, which helps to compress a length of a lens group of the optical lens 100 that is close to the object side of the optical axis along the optical axis O, thereby reducing the thickness of the entire optical lens 100 in a direction of the optical axis close to the object side, and also helps to reduce a size of a head of the first lens L1. An object side surface S1 of the first lens L1 may be convex or concave near the optical axis O, thereby adjusting a surface shape of the object side surface S1 of the first lens L1, which enhances a light path control ability of a light entry surface of the first lens L1. An imaging side surface S2 of the first lens L1 may be concave near the optical axis O, which may also adjust a surface shape of the imaging side surface S2 of the first lens L1, thereby helping to correct aberrations such as astigmatism.

In some embodiments, the second lens L2 may have positive refractive power, thereby balancing the aberrations produced by compressing the lens group of the optical lens 100. An object side surface S3 of the second lens L2 may be convex near the optical axis O, and an imaging side surface S4 of the second lens L2 may be convex or concave near the optical axis O, which may adjust a direction of light travel, thereby helping to balance the volume configuration of the lens group of the optical lens 100 on the object side, and also balance the aberrations produced by the first lens L1, thereby improving the imaging quality of the optical lens 100.

In some embodiments, the third lens L3 may have positive refractive power, thereby cooperating with the second lens L2 and the first lens L1 to reduce the coma aberration in the peripheral field of view. An object side surface S5 of the third lens L3 may be convex near the optical axis O, and an imaging side surface S6 of the third lens L3 may be convex or concave near the optical axis O, which may adjust a surface shape of the third lens L3, thereby adjusting the direction of light travel and helping to increase a size of an image plane.

In some embodiments, the fourth lens L4 may have negative refractive power, thereby balancing the aberrations produced by the optical lens. An object side surface S7 of the fourth lens L4 may be concave near the optical axis O, and an imaging side surface S8 of the fourth lens L4 may be convex or concave near the optical axis O, which may adjust a surface shape of the fourth lens L4, thereby enhancing the focusing performance in the central field of view.

It can be understood that the present application only provides a preferred solution for the refractive power and surface shape design of each lens of the optical lens 100. In some embodiments, the surface shape design and refractive power distribution of the optical lens 100 may also adopt other schemes. Due to space limitations, they are not all listed here, and any combination is also feasible.

It can be understood that the filter IR is arranged between the first lens L1 and the second lens L2. The filter IR may be an infrared cut-off filter. By using an infrared cut-off filter, infrared light can be filtered out, thereby improving the imaging quality and making the image more in line with human visual experience. It can be understood that the filter IR may be made of optical glass with a coating, colored glass, or other materials, and the specific choice can be made according to actual needs, and no specific limitations are made in this embodiment.

In some embodiments, the optical lens 100 may further include an aperture STO, which may be an aperture stop and/or a field stop. It may be arranged between the filter IR and the object side surface S3 of the second lens L2. Thus, it may achieve aperture changes from a large aperture to a small aperture, and realize the aperture adjustment of the optical lens 100. It can be understood that in other embodiments, the aperture STO may be arranged between other two lenses, for example, the aperture STO may be arranged between the second lens L2 and the third lens L3, and the specific choice can be made according to actual needs, and no specific limitations are made in this embodiment.

In some embodiments, the optical lens 100 may further include a protective glass CG, which can be arranged between the imaging side surface S8 of the fourth lens L4 and an image plane IMG of the optical lens 100.

In some embodiments, the optical lens 100 may be applied to endoscopes. Therefore, the materials of the first lens L1, the second lens L2, the third lens L3 and the fourth lens L4 may be selected as plastic, which achieve the lightweight of the optical lens 100 and allow the process for the complex surface shapes of the lenses to be easier.

In some embodiments, the optical lens 100 may satisfy the following conditional expression: 110deg<FOV<155deg, wherein FOV is the maximum field of view angle of the optical lens 100. When the optical lens 100 satisfies the conditional expression 120deg<FOV<155deg, the optical lens 100 may have the characteristic of a large field of view angle, thereby achieving large field of view angle imaging. Further, the optical lens 100 may satisfy the following conditional expression: 120deg<FOV<150deg, thereby allowing the characteristic of the large field of view angle of the optical lens to be more prominent.

In some embodiments, the optical lens 100 may satisfy the following conditional expression: 26deg<FOV/FNO<40deg, wherein FNO is an aperture number of the optical lens 100. In this way, the optical lens 100 may have the characteristics of both a large field of view angle and a large aperture, that is, it may achieve a balance between illumination and depth of field, and increase the amount of light to improve imaging quality. Further, the optical lens 100 may satisfy the following conditional expression: 28deg<FOV/FNO<38deg.

In some embodiments, the optical lens 100 may satisfy the following conditional expression: 3<FNO<5. Thus, the optical lens 100 may have sufficient light intake and meet the large aperture characteristic. Further, the optical lens 100 may satisfy the following conditional expression: 3.4<FNO<4.7, thereby allowing the large aperture characteristic of the optical lens to be more obvious.

In some embodiments, the optical lens 100 may satisfy the following conditional expression: 2.2<TTL/BL<3, wherein BL is a distance from the imaging side surface S8 of the fourth lens L4 to the image plane IMG of the optical lens 100 along the optical axis O (i.e., a back focal length of the optical lens), and TTL is a distance from the object side surface S1 of the first lens L1 to the image plane IMG of the optical lens 100 along the optical axis O (i.e., a total length of the optical lens 100). When the optical lens 100 satisfies the conditional expression 2.2<TTL/BL<3, it may effectively control the back focal length of the optical lens 100 and avoid the situation where the back focal length of the optical lens 100 is too large, resulting in the compression of the lens space of the optical lens 100. Further, the optical lens 100 may satisfy the following conditional expression: 2.4<TTL/BL<2.8, thereby allowing the back focal length of the optical lens 100 to be appropriate and ensuring that the optical lens has sufficient lens space.

In some embodiments, the optical lens 100 may satisfy the following conditional expression: 2.5<TTL/f<5.5, wherein f is a focal length of the optical lens 100. In this way, the miniaturization design of the optical lens 100 may be further achieved while also ensuring that the optical lens 100 has a high imaging quality. Further, the optical lens 100 may satisfy the following conditional expression: 3<TTL/f<5, which is further beneficial for improving the imaging quality of the optical lens.

In some embodiments, the optical lens 100 may satisfy the following conditional expression: 1.7<TTL/ImgH<2.7, wherein ImgH is half of an image height corresponding to the maximum field of view of the optical lens 100. By limiting a ratio of the total length of the optical lens 100 to half of the image height corresponding to the maximum field of view of the optical lens 100, the optical lens 100 may be miniaturized while maintaining good imaging performance. Further, the optical lens 100 may satisfy the following conditional expression: 1.9<TTL/ImgH<2.5, which enables the optical lens to better balance miniaturization and high imaging quality.

In some embodiments, the optical lens 100 may satisfy the following conditional expression: 1.2<ImgH/f<2.3. By limiting a ratio of half of the image height corresponding to the maximum field of view of the optical lens 100 to the focal length of the optical lens 100, a ratio of the height of the optical lens 100 to the image plane IMG of the optical lens 100 may be kept within a smaller range. Thus, through a reasonable structural layout, the miniaturization of the optical lens 100 may be achieved. Further, the optical lens 100 may satisfy the following conditional expression: 1.4<ImgH/f<2.1, which is further beneficial for the miniaturization design of the optical lens 100.

In some embodiments, the optical lens 100 may satisfy the following conditional expression: - 1.4<f1/f<-0.6, wherein f1 is a focal length of the first lens L1 and f is a focal length of the optical lens 100. By controlling a ratio of the focal length of the first lens L1 to the focal length of the optical lens 100, a reasonable refractive power distribution may be achieved for the first lens L1, which is beneficial for light convergence. It also helps to reduce the spherical aberration, chromatic aberration, and distortion of the first lens L1 to a reasonable level, reducing the design difficulty of the subsequent lenses and improving the overall resolution of the optical lens 100. Additionally, it is beneficial for compressing the size of the first lens L1, thereby contributing to the formation of a small-sized optical lens 100. Further, the optical lens 100 may satisfy the following conditional expression: -1.3<f1/f<-0.7, which is further beneficial for compressing the size of the optical lens 100.

In some embodiments, the optical lens 100 may satisfy the following conditional expression: 1.2<f2/f<4, wherein f2 is a focal length of the second lens L2. By controlling a ratio of the focal length of the second lens L2 to the focal length of the optical lens 100, the second lens L2 may have a reasonable refractive power, which may reduce the angle of the light incident from the first lens L1 and improve the overall resolution of the optical lens 100. It also helps to correct the peripheral aberration of the optical lens 100. Further, the optical lens 100 may satisfy the following conditional expression: 1.6<f2/f<3.5, which is further beneficial for improving the imaging quality of the optical lens 100.

In some embodiments, the optical lens 100 may satisfy the following conditional expression: 0.5<f3/f<1.1, wherein f3 is a focal length of the third lens L3. By controlling a ratio of the focal length of the third lens L3 to the focal length of the optical lens 100, the third lens L3 may have a reasonable refractive power, which may further reduce the angle of the light incident from the first lens L1 and improve the overall resolution of the optical lens 100. It also helps to correct the peripheral aberration of the optical lens 100. Further, the optical lens 100 may satisfy the following conditional expression: 0.65<f3/f<0.95, which is further beneficial for improving the imaging quality of the optical lens 100.

In some embodiments, the optical lens 100 may satisfy the following conditional expression: -2<f4/f<-0.8, wherein f4 is a focal length of the fourth lens L4. By controlling a ratio of the focal length of the fourth lens L4 to the focal length of the optical lens 100, the fourth lens L4 may have a reasonable refractive power, which helps correct the aberrations introduced by the first lens L1, the second lens L2, and the third lens L3, thereby improving the imaging quality of the optical lens 100. Further, the optical lens 100 may satisfy the following conditional expression: -1.7<f4/f<-1, which further helps improve the imaging quality of the optical lens 100.

In some embodiments, the optical lens 100 may satisfy the following conditional expression: 0.7<|R11|/f, wherein R11 is a radius of curvature of the object side surface S1 of the first lens L1 at the optical axis O. When the optical lens 100 satisfies the conditional expression 0.7<|R11|/f, it may reduce the complexity of the surface shape of the first lens L1, thereby effectively suppressing the increase of field curvature and distortion, and at the same time reducing the molding difficulty of the first lens L1 and facilitating the control of the size of the first lens L1.

In some embodiments, the optical lens 100 may satisfy the following conditional expression: 0.3<R12/f, wherein R12 is a radius of curvature of the imaging side surface S2 of the first lens L1 at the optical axis O. When the optical lens 100 satisfies the conditional expression 0.3< R12/f, it may reduce the complexity of the surface shape of the first lens L1, thereby effectively suppressing the increase of field curvature and distortion, and at the same time reducing the molding difficulty of the first lens L1. Further, the optical lens 100 may satisfy the following conditional expression: 0.4<R12/f<5, which is more conducive to the molding of the first lens L1.

In some embodiments, the optical lens 100 may satisfy the following conditional expression: 0.6<R21/f<2, wherein R21 is a radius of curvature of the object side surface S3 of the second lens L2 at the optical axis O. When the optical lens 100 satisfies the conditional expression, it may reduce the complexity of the surface shape of the second lens L2, thereby effectively suppressing the increase of field curvature and distortion, and at the same time reducing the molding difficulty of the second lens L2. Further, the optical lens 100 may satisfy the following conditional expression: 0.8<R21/f<1.7, further reducing the molding difficulty of the second lens L2.

In some embodiments, the optical lens 100 may satisfy the following conditional expression: 9<|R22|/f, wherein R22 is a radius of curvature of the imaging side surface S4 of the second lens L2 at the optical axis O. When the optical lens 100 satisfies the conditional expression, it may reduce the complexity of the surface shape of the second lens L2, thereby effectively suppressing the increase of field curvature and distortion, and at the same time reducing the molding difficulty of the second lens L2.

In some embodiments, the optical lens 100 may satisfy the following conditional expression: 0.5<R31/f <1.0, wherein R31 is a radius of curvature of the object side surface of the third lens L3 at the optical axis. When the optical lens 100 satisfies the conditional expression, it may reduce the complexity of the surface shape of the third lens L3, thereby effectively suppressing the increase of field curvature and distortion, and at the same time reducing the molding difficulty of the third lens L3. Further, the optical lens 100 may satisfy the following conditional expression: 0.6<R31/f<0.95, further facilitating the reduction of the molding difficulty of the third lens L3.

In some embodiments, the optical lens 100 may satisfy the following conditional expression: - 1.1<R32/<-0.4, wherein R32 is a radius of curvature of the imaging side surface of the third lens L3 at the optical axis. When the optical lens 100 satisfies the conditional expression, it may reduce the complexity of the surface shape of the third lens L3, thereby effectively suppressing the increase of field curvature and distortion, and at the same time reducing the molding difficulty of the third lens L3. Further, the optical lens 100 may satisfy the following conditional expression: -0.95<R32/f<-0.5, further facilitating the reduction of the molding difficulty of the third lens L3.

In some embodiments, the optical lens 100 may satisfy the following conditional expression: - 1.3<R41/f<-0.4, wherein R41 is a curvature radius of the object side surface S7 of the fourth lens L4 at the optical axis. When the optical lens 100 satisfies the conditional expression, it may reduce the surface complexity of the fourth lens L4, thereby effectively suppressing the increase of field curvature and distortion, and at the same time reducing the molding difficulty of the fourth lens L4. Further, the optical lens 100 may satisfy the following conditional expression: -1.2<R41/f<-0.5, which is beneficial for the molding of the fourth lens L4.

In some embodiments, the optical lens 100 may satisfy the following conditional expression: 3<|R42|/f, wherein R42 is a curvature radius of the imaging side surface S8 of the fourth lens L4 at the optical axis. When the optical lens 100 satisfies the conditional expression, it may reduce the surface complexity of the fourth lens L4, thereby effectively suppressing the increase of field curvature and distortion, and at the same time reducing the molding difficulty of the fourth lens L4.

In some embodiments, the optical lens 100 may satisfy the following conditional expression: - 2.3<R11/R12<100. By limiting a ratio of the curvature radius of the object side surface S1 to the curvature radius of the imaging side surface S2 of the first lens L1 near the optical axis O, the bending situation of the surface shape of the first lens L1 may be reasonably controlled, which is convenient for the processing of the first lens L1 and is conducive to improving the processing yield of the optical lens 100.

In some embodiments, the optical lens 100 may satisfy the following conditional expression: 7<|R22/R21|<30. By limiting a ratio of the curvature radius of the object side surface S3 to the curvature radius of the imaging side surface S4 of the second lens L2 near the optical axis O, the bending situation of the surface shape of the second lens L2 may be reasonably controlled, which is convenient for the processing of the second lens L2 and is conducive to improving the processing yield of the optical lens 100.

In some embodiments, the optical lens 100 may satisfy the following conditional expression: $-1.7<R31/R32<-0.6$. By limiting a ratio of the curvature radius of the object side surface to the curvature radius of the imaging side surface of the third lens L3 near the optical axis O, the bending situation of the surface shape of the third lens L3 may be reasonably controlled, which is convenient for the processing of the third lens L3 and is conducive to improving the processing yield of the optical lens 100. In addition, it may further correct the astigmatism introduced by the first lens L1 and the second lens L2, so that the light of the optical lens 100 may be deflected at a smaller angle. Further, the optical lens 100 may satisfy the following conditional expression: $-1.4 < R31/R32 < -0.8$, which is more conducive to the deflection of light.

In some embodiments, the optical lens 100 may satisfy the following conditional expression: $4<|R42/R41|$. By limiting a ratio of the curvature radius of the object side surface S7 and the imaging side surface S8 of the fourth lens L4 near the optical axis O, the bending situation of the surface shape of the fourth lens L4 may be reasonably controlled, which is convenient for the processing of the fourth lens L4 and is conducive to improving the processing yield of the optical lens 100.

In some embodiments, the optical lens 100 may satisfy the following conditional expression: $0.5<(|SAG11|+|SAG12|)/CT1<1.5$, wherein CT1 is a thickness of the first lens L1 at the optical axis O, SAG11 is a distance from the maximum effective semi-aperture of the object side surface S1 of the first lens L1 to an intersection point of the object side surface S1 of the first lens L1 and the optical axis in the direction of the optical axis (i.e., a vector height of the object side surface S1 of the first lens L1), and SAG12 is a distance from the maximum effective semi-aperture of the imaging side surface S2 of the first lens L1 to an intersection point of the imaging side surface S2 and the optical axis in the direction of the optical axis (i.e., a vector height of the imaging side surface S2 of the first lens L1). When the optical lens 100 satisfies the conditional expression, it can facilitate the control of the refractive power and thickness of the first lens L1, thereby preventing the first lens L1 from being too thick or too thin, and facilitating the processing of the first lens. At the same time, it may reduce the incident angle of the light on the first lens L1, thereby reducing the sensitivity of the optical lens 100. Further, the optical lens 100 may satisfy the following conditional expression: $0.55<(|SAG11|+|SAG12|)/CT1<1.3$, which is more convenient for the processing of the first lens L1.

In some embodiments, the optical lens 100 may satisfy the following conditional expression: $0.1<(|SAG21|+|SAG22|)/CT2<0.4$, wherein CT2 is a thickness of the second lens L2 at the optical axis O, SAG21 is a distance from the maximum effective semi-aperture of the object side surface S3 of the second lens L2 to an intersection point of the object side surface S3 of the second lens L2 and the optical axis in the direction of the optical axis (i.e., a vector height of the object side surface S3 of the second lens L2), and SAG22 is a distance from the maximum effective semi-aperture of the imaging side surface S4 of the second lens L2 to an intersection point of the imaging side surface S4 of the second lens L2 and the optical axis in the direction of the optical axis (i.e., a vector height of the imaging side surface S4 of the second lens L2). When the optical lens satisfies the conditional expression, it is convenient to control the refractive power and thickness of the second lens L2, thereby preventing the second lens L2 from being too thick or too thin, and facilitating the processing of the second lens. At the same time, it may also reduce the incident angle of light on the second lens L2, thereby reducing the sensitivity of the optical lens 100. Further, the optical lens 100 may satisfy the following conditional expression: $0.16<(|SAG21|+|SAG22|)/CT2<0.3$, which is more convenient for the processing of the second lens L2.

In some embodiments, the optical lens 100 may satisfy the following conditional expression: $0.1<(|SAG31|+|SAG32|)/CT3<0.9$, wherein CT3 is a thickness of the third lens L3 at the optical axis O, SAG31 is a distance from the maximum effective semi-aperture of the object side surface S5 of the third lens L3 to an intersection point of the object side surface S5 of the third lens L3 and the optical axis in the direction of the optical axis (i.e., a vector height of the object side surface S5 of the third lens L3), and SAG32 is a distance from the maximum effective semi-aperture of the imaging side surface S6 of the third lens L3 to an intersection point of the imaging side surface S6 of the third lens L3 and the optical axis in the direction of the optical axis (i.e., a vector height of the imaging side surface S6 of the third lens L3). When the optical lens satisfies the conditional expression, it is convenient to control the refractive power and thickness of the third lens L3, thereby preventing the third lens L3 from being too thick or too thin, and facilitating the processing of the third lens L3. At the same time, it is also beneficial for reducing the distortion and field curvature caused by the first lens L1 and the second lens L2, thereby allowing the refractive power near the image plane IMG of the optical lens 100 to be more uniform. Further, the optical lens 100 may satisfy the following conditional expression: $0.2<(|SAG31|+|SAG32|)/CT3<0.8$, which is more convenient for the processing of the third lens L3.

In some embodiments, the optical lens 100 may satisfy the following conditional expression: $0.3<(|SAG41|+|SAG42|)/CT4<1$, wherein CT4 is a thickness of the fourth lens L4 at the optical axis O, SAG41 is a distance from the maximum effective semi-aperture of the object side surface S7 of the fourth lens L4 to an intersection point of the object side surface S7 of the fourth lens L4 and the optical axis in the direction of the optical axis (i.e., a vector height of the object side surface S7 of the fourth lens L4), and SAG42 is a distance from the maximum effective semi-aperture of the imaging side surface S8 of the fourth lens L4 to an intersection point of the imaging side surface S8 of the fourth lens L4 and the optical axis in the direction of the optical axis (i.e., a vector height of the imaging side surface S8 of the fourth lens L4). When the optical lens satisfies the conditional expression, it is convenient to control the thickness and refractive power of the fourth lens L4, allowing the thickness of the fourth lens L4 to be controllable, and thus facilitating the processing and molding of the fourth lens L4. At the same time, it may also effectively reduce the incident angle of light on the image plane IMG of the optical lens 100, thereby reducing the sensitivity of the optical lens 100, and it is also beneficial for correcting the distortion and field curvature caused by the first lens L1, the second lens L2, and the third lens L3, allowing the refractive power near the optical lens 100 to be more uniform. Further, the optical lens 100 may satisfy the following conditional expression: $0.35<(ISAG411+ISAG421)/CT4<0.9$, which is more convenient for the processing of the third lens L3.

In some embodiments, the optical lens 100 may satisfy the following conditional expression: $-14<f4/|SAGY41|<-3$. When the optical lens satisfies the conditional expression, it may allow the refractive power and shape of the fourth lens L4 to be reasonable, thereby minimizing chromatic aberration and spherical aberration to the greatest extent, which is beneficial for improving the imaging quality of the optical lens. At the same time, through reasonable refractive power distribution, it may enhance the light-gathering ability of the optical lens 100 and is conducive to controlling the size of the optical lens 100. Further, the optical lens 100 may satisfy the following conditional expression: $-12<f4/|SAGY41|<-4$, which is further conducive to controlling the size of the optical lens 100.

In some embodiments, the optical lens 100 may satisfy the following conditional expression: $f4/|SAGY42|<-10$. When the optical lens satisfies the conditional expression, it may allow the refractive power and shape of the fourth lens L4 to be reasonable, thereby minimizing chromatic aberration and spherical aberration to the greatest extent, which is beneficial for improving the imaging quality of the optical lens. At the same time, through reasonable refractive power distribution, it may enhance the light-gathering ability of the optical lens 100 and is conducive to controlling the size of the optical lens 100. Further, the optical lens 100 may satisfy the following conditional expression: $f4/|SAGY42|<-16$, which is further conducive to controlling the size of the optical lens 100.

In some embodiments, the optical lens 100 may satisfy the following conditional expression: $1<ET1/CT1<1.9$, wherein ET1 is a distance from the maximum effective semi-aperture of the object side surface S1 of the first lens L1 to the maximum effective semi-aperture of the imaging side surface S2 of the first lens L1 (i.e., an edge thickness of the first lens L1). By limiting a ratio of the edge thickness of the first lens L1 to the center thickness of the first lens L1, the thickness of the first lens L1 may be reasonably controlled, thereby achieving a miniaturized design of the optical lens 100, and at the same time, it is beneficial for reducing the sensitivity of the optical lens 100. Further, the optical lens 100 may satisfy the following conditional expression: $1.2<ET1/CT1<1.7$, which is beneficial for the miniaturized design of the optical lens 100.

In some embodiments, the optical lens 100 may satisfy the following conditional expression: $1<ET4/CT4<2.1$, wherein ET4 is a distance from the maximum effective semi-aperture of the object side surface S7 of the fourth lens L4 to the maximum effective semi-aperture of the imaging side surface S8 of the fourth lens L4 (i.e., an edge thickness of the fourth lens L4). By limiting a ratio of the edge thickness of the fourth lens L4 to the center thickness of the fourth lens L4, the thickness of the fourth lens L4 may be reasonably controlled, thereby achieving a miniaturized design of the optical lens 100, and at the same time, it is beneficial for reducing the sensitivity of the optical lens 100. Further, the optical lens 100 may satisfy the following conditional expression: $1.2<ET4/CT4<1.9$, which is beneficial for the miniaturized design of the optical lens 100.

In some embodiments, the optical lens 100 may satisfy the following conditional expression: $0.9<CT3/CT4<2.1$, wherein CT3 is a thickness of the third lens L3 at the optical axis (i.e., a center thickness of the third lens L3), and CT4 is a thickness of the fourth lens L4 at the optical axis (i.e., a center thickness of the fourth lens L4). When the above conditional expression is satisfied, a thickness ratio of the third lens L3 and the fourth lens L4 can be effectively controlled, achieving a miniaturized design of the optical lens 100, and at the same time, the third lens L3 and the fourth lens L4 may have better processing characteristics, which is beneficial for reducing the sensitivity of the optical lens 100. Further, the optical lens 100 may satisfy the following conditional expression: $1.1<CT3/CT4<1.9$, which is beneficial for the miniaturized design of the optical lens 100.

In some embodiments, the optical lens 100 may satisfy the following conditional expression: $2.5<TD/\Sigma AT<3.5$, wherein TD is a distance from the object side surface of the first lens L1 to the imaging side surface of the fourth lens L4 at the optical axis, and $\Sigma AT$ is a sum of distances from the imaging side surface of the first lens L1 to an object side surface of the filter IR, from an imaging side surface of the filter IR to the object side surface of the second lens L2, from the imaging side surface of the second lens L2 to the object side surface of the third lens L3, and from the imaging side surface of the third lens L3 to the object side surface of the fourth lens L4 at the optical axis. When the above conditional expression is satisfied, the thickness of each lens and the spacing between two adjacent lenses can be further balanced, thereby allowing the arrangement of the lenses in the optical lens 100 to be more compact and further reducing the total length of the optical lens 100, which is beneficial for the miniaturization design of the optical lens. Further, the optical lens 100 may satisfy the following conditional expression: $2.6<TD/\Sigma AT<3$, thereby allowing the overall structure of the optical lens 100 to be more compact and more conducive to miniaturization design.

In some embodiments, the optical lens 100 may satisfy the following conditional expression: $6<AT12/AT34<22$, wherein AT12 is a sum of distances from the imaging side surface of the first lens to the object side surface of the filter and from the imaging side surface of the filter to the object side surface of the second lens at the optical axis, and AT34 is a distance from the imaging side surface of the third lens to the object side surface of the fourth lens at the optical axis. When the above conditional expression is satisfied, the arrangement between the third lens L3 and the fourth lens L4 is more compact, while the arrangement between the first lens L1 and the second lens L2 is relatively loose, which is beneficial for controlling the small-angle deflection of light between the first lens and the second lens and facilitating the light collection of the optical lens. Further, the optical lens 100 may satisfy the following conditional expression: $6.3<AT12/AT34<21.9$.

In some embodiments, the optical lens 100 may satisfy the following conditional expression: $1<SD11/SD42<1.3$, wherein SD11 is the maximum effective semi-aperture of the object side surface of the first lens, and SD42 is the maximum effective semi-aperture of the imaging side surface of the fourth lens. When the above conditional expression is satisfied, the aperture of the object side surface of the first lens L1 and the aperture of the imaging side surface of the fourth lens L4 are close, which is beneficial for improving the space utilization rate of the optical lens 100.

In some embodiments, the optical lens 100 may satisfy the following conditional expression: $0.8<SD22/SD31<1$, wherein SD22 is the maximum effective semi-aperture of the imaging side surface of the second lens, and SD31 is the maximum effective semi-aperture of the object side surface of the third lens. When the above conditional expression is satisfied, the aperture of the imaging side surface of the second lens L2 and the aperture of the object side surface of the third lens L3 are close, which can reduce a step difference between them and allow the transition of light between them to be smoother.

In some embodiments, the optical lens 100 may satisfy the following conditional expression: $0.4<SD11/ImgH<0.6$, thereby allowing the diameter of the object side surface of the first lens L1 to be slightly smaller than the size of the image plane IMG, which may reduce a head diameter of the optical lens and achieve a small head design.

The optical lens 100 of the present disclosure will be described in detail below with reference to specific parameters.

First embodiment

FIG. 1A is a schematic structural diagram of the optical lens 100 of the first embodiment, the optical lens 100 sequentially includes a first lens L1, a filter IR, an aperture STO, a second lens L2, a third lens L3, a fourth lens L4, and a protective glass CG.

In the illustrated embodiment, the first lens L1 has negative refractive power, an object side surface S1 and an imaging side surface S2 of the first lens L1 are both concave near the optical axis O. The second lens L2 has positive refractive power, an object side surface S3 and an imaging side surface S4 of the second lens L2 are both convex near the optical axis O. The third lens L3 has positive refractive power, an object side surface S5 and an imaging side surface S6 of the third lens L3 are both convex near the optical axis O. The fourth lens L4 has negative refractive power, an object side surface S7 and an imaging side surface S8 of the fourth lens L4 are respectively concave and convex near the optical axis O.

The parameters of the optical lens 100 are given in Table 1 below. The components from the object side to the image side along the optical axis of the optical lens 100 are arranged in the order from top to bottom in Table 1. In the same lens, a surface with a smaller surface number is the object side surface of the lens, and a surface with a larger surface number is the imaging side surface of the lens. For example, surface numbers 1 and 2 correspond to the object side surface S1 and imaging side surface S2 of the first lens L1, respectively. The radius Y in Table 1 is the radius of curvature of the object side surface or the imaging side surface with the corresponding surface number at the optical axis. The first value in the "Thickness" parameter column of the lens is the thickness of the lens at the optical axis, and the second value is a distance from the imaging side surface of the lens to a rear surface in an imaging side direction on the optical axis. The value of the aperture STO in the "Thickness" parameter column is a distance from the aperture STO to a vertex of a latter surface (the vertex refers to an intersection of the surface and the optical axis) on the optical axis, and by default, a direction from the object side surface of the first lens L1 to the imaging side surface of the last lens is a positive direction of the optical axis. When the value of the aperture STO in the "Thickness" parameter column is negative, it indicates that the aperture STO is arranged on the image side of the vertex of the latter surface. When the value of the aperture STO in the "Thickness" parameter column is a positive value, the aperture STO is arranged on the object side of the vertex of the latter surface. It can be understood that the units of the Y radius, the thickness, and the focal length in Table 1 are all mm. The refractive index and Abbe number in Table 1 are obtained at the reference wavelength of 587.6 nm, and the focal length is obtained at the reference wavelength of 555 nm.

In the first embodiment, the object side surfaces and the imaging side surfaces of the first lens L1 to the seventh lens L7 are both aspheric surfaces. The surface shape x of the aspheric surface can be defined by, but not limited to, the following aspherical formula:

$$x = \frac{ch^2}{1+\sqrt{1-(K+1)c^2h^2}} + \sum Aih^i$$

Wherein, x is a height distance along the optical axis from a position of the aspheric surface at height h to the vertex of the aspheric surface; c is a curvature of the vertex of the aspheric surface, and c=1/Y (that is, the curvature of the vertex of the aspheric surface c is the reciprocal of the Y radius in the following Table 1); K is the cone coefficient; Ai is the coefficient corresponding to the i-th higher-order term of the aspheric surface. Table 2 shows the higher-order term coefficients A4, A6, A8, A10, A12, A14, A16, A18, and A20 of the aspheric surfaces of the first lens L1 to the seventh lens L7.

It can be understood that the surface numbers 1 and 2 in Table 1 and Table 2 respectively correspond to the object side surface S1 and imaging side surface S2 of the first lens L1, while surface numbers 3 and 4 respectively correspond to the object side surface S3 and imaging side surface S4 of the second lens L2, by parity of reasoning, surface numbers 7 and 8 respectively correspond to the object side surface S7 and imaging side surface S8 of the fourth lens L4.

Table 1

| First embodiment | | | | | | | | |
|---|---|---|---|---|---|---|---|---|
| f=0.675mm, FNO=4.4, FOV=132.1deg, TTL=2.25mm | | | | | | | | |
| Surface number | Surface name | Surface type | Y radius | Thickness | Material | Refractive index | Abbe number | Focal length |
| OBJ | Object side | sphere | infinity | 10 | | | | |
| 1 | First lens | asphere | -0.810 | 0.2 | plastic | 1.538 | 56.01 | -0.575 |
| 2 | | asphere | 0.544 | 0.142 | | | | |
| IR | Filter | sphere | infinity | 0.21 | glass | | | |
| | | sphere | infinity | 0.012 | | | | |
| STO | Aperture | sphere | infinity | 0.001 | | | | |
| 3 | Second lens | asphere | 0.647 | 0.296 | plastic | 1.537 | 55.75 | 1.143 |
| 4 | | asphere | -10 | 0.033 | | | | |
| 5 | Third lens | asphere | 0.527 | 0.364 | plastic | 1.546 | 56.11 | 0.489 |
| 6 | | asphere | -0.408 | 0.033 | | | | |
| 7 | Fourth lens | asphere | -0.411 | 0.21 | plastic | 1.667 | 20.38 | -0.754 |
| 8 | | asphere | -2.707 | 0.49734852 1 | | | | |
| CG | Protective glass | sphere | infinity | 0.4 | glass | | | |
| | | sphere | infinity | 0.0324 | | | | |
| IMG | Imaging surface | sphere | infinity | 0 | | | | |

Table 2

| First embodiment | | | | | |
|---|---|---|---|---|---|
| Surface number | K | A4 | A6 | A8 | A10 |
| 1 | -5.61257E+01 | 2.80839E+00 | -6.36212E+00 | -1.67402E+02 | 2.64316E+03 |
| 2 | 8.27371E-01 | 2.22067E+01 | -2.51327E+02 | -3.96109E+04 | 4.62871E+06 |
| 3 | -1.68737E+01 | 7.98736E+00 | -8.81395E+02 | 2.47557E+05 | -4.40563E+07 |
| 4 | -9.90000E+01 | -3.34324E+01 | 7.42135E+02 | -1.06540E+04 | -3.54227E+05 |
| 5 | -2.55462E+01 | -1.14293E+01 | 3.90018E+01 | 2.59953E+03 | -2.59887E+05 |
| 6 | -2.53897E-01 | 9.65014E+00 | -1.73075E+02 | -2.52243E+03 | 1.72831E+05 |
| 7 | -2.83878E-01 | 1.38086E+01 | -3.05429E+02 | 5.97216E+02 | 1.03570E+05 |
| 8 | 5.32685E+00 | 5.40352E+00 | -9.18311E+01 | 9.09615E+02 | -5.24879E+03 |

(continued)

| Surface number | A12 | A14 | A16 | A18 | A20 |
|---|---|---|---|---|---|
| 1 | -1.97823E+04 | 8.75135E+04 | -2.32363E+05 | 3.42747E+05 | -2.16105E+05 |
| 2 | -2.31387E+08 | 6.52848E+09 | -1.06798E+11 | 9.45592E+11 | -3.50478E+12 |
| 3 | 4.59880E+09 | -2.89024E+11 | 1.07499E+13 | -2.17069E+14 | 1.82208E+15 |
| 4 | 2.41479E+07 | -6.27362E+08 | 8.75975E+09 | -6.42154E+10 | 1.91983E+11 |
| 5 | 1.02082E+07 | -2.36018E+08 | 3.21197E+09 | -2.39302E+10 | 7.58843E+10 |
| 6 | -3.67979E+06 | 4.38127E+07 | -3.12068E+08 | 1.24290E+09 | -2.11122E+09 |
| 7 | -2.34359E+06 | 2.62448E+07 | -1.73632E+08 | 6.57824E+08 | -1.10171E+09 |
| 8 | 1.53979E+04 | -5.29543E+03 | -1.05430E+05 | 2.97618E+05 | -2.68510E+05 |

Referring to (A) of FIG. 2, (A) of FIG. 2 is the longitudinal spherical aberration diagram of the optical lens 100 in the first embodiment at wavelengths of 435nm, 470nm, 510nm, 555nm, 610nm and 650nm. In (A) of FIG. 2, the abscissa along the X-axis direction represents the deviation of the focus point in mm, and the ordinate along the Y-axis direction represents the normalized field of view. It can be seen from (A) of FIG. 2 that the spherical aberration value of the optical lens 100 in the first embodiment is well, indicating that the imaging quality of the optical lens 100 in this embodiment is well.

Referring to (B) of FIG. 2, (B) of FIG. 2 is an astigmatism curve diagram of the optical lens 100 in the first embodiment at a wavelength of 555 nm. Wherein, the abscissa along the X-axis direction represents the deviation of the focus point in mm, and the ordinate along the Y-axis direction represents the image height in mm. T in the astigmatism curve diagram represents the curvature of the image plane IMG in the tangential direction, and S represents the curvature of the image plane IMG in the sagittal direction. It can be seen from (B) of FIG. 2 that at this wavelength, the astigmatism of the optical lens 100 has been well compensated.

Referring to (C) of FIG. 2, (C) of FIG. 2 is a distortion diagram of the optical lens 100 in the first embodiment at a wavelength of 555nm. Wherein, the abscissa along the X-axis direction represents the distortion, and the ordinate along the Y-axis direction represents the image height in mm. It can be seen from (C) of FIG. 2 that at this wavelength, the distortion of the optical lens 100 has been well corrected.

Second embodiment

Referring to FIG. 3, the optical lens 100 sequentially includes a first lens L1, a filter IR, an aperture STO, a second lens L2, a third lens L3, a fourth lens L4, and a protective glass CG.

In the illustrated embodiment, the first lens L1 has negative refractive power, an object side surface S1 and an imaging side surface S2 of the first lens L1 are both concave near the optical axis O. The second lens L2 has positive refractive power, an object side surface S3 and an imaging side surface S4 of the second lens L2 are respectively convex and concave near the optical axis O. The third lens L3 has positive refractive power, an object side surface S5 and an imaging side surface S6 of the third lens L3 are respectively convex and concave near the optical axis O. The fourth lens L4 has negative refractive power, an object side surface S7 and an imaging side surface S8 of the fourth lens L4 are respectively concave and convex near the optical axis O.

The parameters of the optical lens 100 are given in Table 3 below. The definitions of each parameter can be obtained from the description of the previous embodiments, which will not be repeated here. Correspondingly, Table 4 below shows the high-order coefficients of the aspheric lenses that can be used in the second embodiment.

Table 3

| Second embodiment | | | | | | | | |
|---|---|---|---|---|---|---|---|---|
| f=0.5875mm, FNO=3.8, FOV=139.5deg, TTL=2.4mm | | | | | | | | |
| Surface number | Surface name | Surface type | Y radius | Thickness | Material | Refractive index | Abbe number | Focal length |
| OBJ | Object side | sphere | infinity | 10 | | | | |
| 1 | First lens | asphere | -1.033 | 0.19 | plastic | 1.538 | 56.01 | -0.569 |
| 2 | | asphere | 0.463 | 0.164 | | | | |

(continued)

| Second embodiment | | | | | | | | |
|---|---|---|---|---|---|---|---|---|
| f=0.5875mm, FNO=3.8, FOV=139.5deg, TTL=2.4mm | | | | | | | | |
| Surface number | Surface name | Surface type | Y radius | Thickness | Material | Refractive index | Abbe number | Focal length |
| IR | Filter | sphere | infinity | 0.21 | glass | | | |
| | | sphere | infinity | 0.02 | | | | |
| STO | Aperture | sphere | infinity | -0.007 | | | | |
| 3 | Second lens | asphere | 0.717 | 0.338 | plastic | 1.537 | 55.75 | 1.482 |
| 4 | | asphere | 6.105 | 0.031 | | | | |
| 5 | Third lens | asphere | 0.449 | 0.318 | plastic | 1.546 | 56.11 | 0.436 |
| 6 | | asphere | -0.379 | 0.03 | | | | |
| 7 | Fourth lens | asphere | -0.396 | 0.21 | plastic | 1.667 | 20.38 | -0.809 |
| 8 | | asphere | -1.806 | 0.453 | | | | |
| CG | Protective glass | sphere | infinity | 0.4 | glass | | | |
| | | sphere | infinity | 0.0324 | | | | |
| IMG | Imaging surface | sphere | infinity | 0 | | | | |

Table 4

| Second embodiment | | | | | |
|---|---|---|---|---|---|
| Surface number | K | A4 | A6 | A8 | A10 |
| 1 | -9.90000E+01 | 4.09580E+00 | -8.05047E+00 | -2.93327E+02 | 4.35532E+03 |
| 2 | 9.99020E-01 | 3.08018E+01 | -2.20360E+03 | 1.84155E+05 | -9.78388E+06 |
| 3 | -1.73301E+01 | 4.77364E+00 | 9.49144E+00 | 6.60847E+03 | -4.53574E+06 |
| 4 | -1.62529E+01 | -3.77357E+01 | 1.15749E+03 | -3.73933E+04 | 7.80083E+05 |
| 5 | -1.75219E+01 | -1.33361E+01 | 4.74542E+02 | -3.05107E+04 | 1.31194E+06 |
| 6 | -1.61959E-01 | 8.10148E+00 | 1.24618E+02 | -1.84377E+04 | 6.11199E+05 |
| 7 | -3.56795E-01 | 1.16141E+01 | -5.32212E+01 | -1.17312E+04 | 4.27877E+05 |
| 8 | -1.50996E+01 | 4.83197E+00 | -7.55766E+01 | 7.95762E+02 | -5.76690E+03 |
| Surface number | A12 | A14 | A16 | A18 | A20 |
| 1 | -3.10983E+04 | 1.30962E+05 | -3.30069E+05 | 4.61109E+05 | -2.74980E+05 |
| 2 | 3.29368E+08 | -6.98508E+09 | 9.05814E+10 | -6.55224E+11 | 2.02440E+12 |
| 3 | 6.51969E+08 | -4.68749E+10 | 1.83875E+12 | -3.71114E+13 | 2.98914E+14 |
| 4 | -6.39438E+06 | -1.18423E+08 | 3.85262E+09 | -4.13978E+10 | 1.65687E+11 |
| 5 | -3.80576E+07 | 7.18924E+08 | -8.53260E+09 | 5.76623E+10 | -1.70747E+11 |
| 6 | -1.08401E+07 | 1.16683E+08 | -7.72141E+08 | 2.91271E+09 | -4.81812E+09 |
| 7 | -7.40229E+06 | 7.52312E+07 | -4.65538E+08 | 1.64857E+09 | -2.58695E+09 |
| 8 | 3.03373E+04 | -1.18961E+05 | 3.30402E+05 | -5.60246E+05 | 4.20392E+05 |

Referring to FIG. 4, it can be seen from (A) a longitudinal spherical aberration diagram, (B) an astigmatism curve diagram, and (C) a distortion diagram in FIG. 4 that, the spherical aberration value, astigmatism, and distortion of the optical lens 100 are well controlled, so that the optical lens 100 of this embodiment has a good imaging quality. In addition, regarding the wavelengths corresponding to the curves in (A) in Figure 4, (B) in Figure 4, and (C) in Figure 4, please refer to the contents described in (A) in Figure 2, (B) in Figure 2, and (C) in Figure 2 in the first embodiment and will not be described again here.

Third embodiment

Referring to FIG. 5, the optical lens 100 sequentially includes a first lens L1, a filter IR, an aperture STO, a second lens L2, a third lens L3, a fourth lens L4, and a protective glass CG.

In the illustrated embodiment, the first lens L1 has negative refractive power, an object side surface S1 and an imaging side surface S2 of the first lens L1 are both concave near the optical axis O. The second lens L2 has positive refractive power, an object side surface S3 and an imaging side surface S4 of the second lens L2 are respectively convex and concave near the optical axis O. The third lens L3 has positive refractive power, an object side surface S5 and an imaging side surface S6 of the third lens L3 are both convex near the optical axis O. The fourth lens L4 has negative refractive power, an object side surface S7 and an imaging side surface S8 of the fourth lens L4 are both concave near the optical axis O.

The parameters of the optical lens 100 are given in Table 5 below. The definitions of each parameter can be obtained from the description of the previous embodiments, which will not be repeated here. Correspondingly, Table 6 below shows the high-order coefficients of the aspheric lenses that can be used in the third embodiment.

Table 5

| Third embodiment | | | | | | | | |
|---|---|---|---|---|---|---|---|---|
| f=0.621mm, FNO=3.8, FOV=130deg, TTL=2.38mm | | | | | | | | |
| Surface number | Surface name | Surface type | Y radius | Thickness | Material | Refractive index | Abbe number | Focal length |
| OBJ | Object side | sphere | infinity | 10 | | | | |
| 1 | First lens | asphere | -0.868 | 0.182 | plastic | 1.538 | 56.01 | *-0.555* |
| 2 | | asphere | 0.489 | 0.183 | | | | |
| IR | Filter | sphere | infinity | 0.21 | glass | | | |
| | | sphere | infinity | 0.023 | | | | |
| STO | Aperture | sphere | infinity | -0.013 | | | | |
| 3 | Second | asphere | 0.685 | 0.302 | plastic | 1.537 | 55.75 | 1.335 |
| 4 | lens | asphere | 13.357 | 0.030 | | | | |
| 5 | Third lens | asphere | 0.443 | 0.281 | plastic | 1.546 | 56.11 | 0.477 |
| 6 | | asphere | -0.489 | 0.03 | | | | |
| 7 | Fourth lens | asphere | -0.572 | 0.21 | plastic | 1.667 | 20.38 | -0.811 |
| 8 | | asphere | 11.352 | 0.483 | | | | |
| CG | Protective glass | sphere | infinity | 0.4 | glass | | | |
| | | sphere | infinity | 0.0324 | | | | |
| IMG | Imaging surface | sphere | infinity | 0 | | | | |

Table 6

| Third embodiment | | | | | |
|---|---|---|---|---|---|
| Surface number | K | A4 | A6 | A8 | A10 |
| 1 | -7.57994E+01 | 3.44692E+00 | 5.34746E+00 | -4.79901E+02 | 5.80434E+03 |
| 2 | 8.06148E-01 | 3.06487E+01 | -2.04173E+03 | 1.62216E+05 | -8.00863E+06 |
| 3 | -1.94719E+01 | 5.78955E+00 | -2.37325E+02 | 2.36953E+04 | -3.21621E+06 |
| 4 | -9.90000E+01 | -4.02716E+01 | 1.23613E+03 | -4.21612E+04 | 9.94614E+05 |
| 5 | -1.73663E+01 | -1.34032E+01 | 7.24632E+02 | -5.75219E+04 | 2.84963E+06 |

(continued)

| Third embodiment | | | | | |
|---|---|---|---|---|---|
| Surface number | K | A4 | A6 | A8 | A10 |
| 6 | -7.85129E-01 | 1.02677E+01 | -3.48840E+01 | -8.60175E+03 | 2.22786E+05 |
| 7 | 7.36458E-02 | 8.79170E+00 | 1.00851E+01 | -1.19932E+04 | 3.64909E+05 |
| 8 | -9.90000E+01 | 2.80176E+00 | -1.93564E+01 | -4.98528E+02 | 1.26415E+04 |
| Surface number | A12 | A14 | A16 | A18 | A20 |
| 1 | -3.79655E+04 | 1.51034E+05 | -3.64850E+05 | 4.93100E+05 | -2.86439E+05 |
| 2 | 2.46665E+08 | -4.75697E+09 | 5.59934E+10 | -3.68229E+11 | 1.03867E+12 |
| 3 | 2.49890E+08 | -1.14892E+10 | 3.07504E+11 | -4.34481E+12 | 2.46004E+13 |
| 4 | -1.44830E+07 | 1.02968E+08 | -3.33928E+07 | -3.61810E+09 | 1.48240E+10 |
| 5 | -8.98742E+07 | 1.79158E+09 | -2.18740E+10 | 1.48595E+11 | -4.27113E+11 |
| 6 | -1.41136E+06 | -2.13635E+07 | 4.13635E+08 | -2.59465E+09 | 5.90841E+09 |
| 7 | -4.91271E+06 | 3.14663E+07 | -6.10354E+07 | -2.62287E+08 | 1.05410E+09 |
| 8 | -1.30058E+05 | 7.39131E+05 | -2.40820E+06 | 4.20669E+06 | -3.05237E+06 |

Referring to FIG. 6, it can be seen from (A) a longitudinal spherical aberration diagram, (B) an astigmatism curve diagram, and (C) a distortion diagram in FIG. 6 that, the spherical aberration value, astigmatism, and distortion of the optical lens 100 are well controlled, so that the optical lens 100 of this embodiment has a good imaging quality. In addition, regarding the wavelengths corresponding to the curves in (A) in Figure 6, (B) in Figure 6, and (C) in Figure 6, please refer to the contents described in (A) in Figure 2, (B) in Figure 2, and (C) in Figure 2 in the first embodiment and will not be described again here.

Fourth embodiment

Referring to FIG. 7, the optical lens 100 sequentially includes a first lens L1, a filter IR, an aperture STO, a second lens L2, a third lens L3, a fourth lens L4, and a protective glass CG.

In the illustrated embodiment, the first lens L1 has negative refractive power, an object side surface S1 and an imaging side surface S2 of the first lens L1 are both concave near the optical axis O. The second lens L2 has positive refractive power, an object side surface S3 and an imaging side surface S4 of the second lens L2 are respectively convex and concave near the optical axis O. The third lens L3 has positive refractive power, an object side surface S5 and an imaging side surface S6 of the third lens L3 are both convex near the optical axis O. The fourth lens L4 has negative refractive power, an object side surface S7 and an imaging side surface S8 of the fourth lens L4 are both concave near the optical axis O.

The parameters of the optical lens 100 are given in Table 7 below. The definitions of each parameter can be obtained from the description of the previous embodiments, which will not be repeated here. Correspondingly, Table 8 below shows the high-order coefficients of the aspheric lenses that can be used in the fourth embodiment.

Table 7

| Fourth embodiment | | | | | | | | |
|---|---|---|---|---|---|---|---|---|
| f=0.621mm, FNO=3.8, FOV=130deg, TTL=2.38mm | | | | | | | | |
| Surface number | Surface name | Surface type | Y radius | Thickness | Material | Refractive index | Abbe number | Focal length |
| OBJ | Object side | sphere | infinity | 10 | | | | |
| 1 | First lens | asphere | -0.868 | 0.182 | plastic | 1.538 | 56.01 | *-0.555* |
| 2 | | asphere | 0.489 | 0.183 | | | | |
| IR | Filter | sphere | infinity | 0.21 | glass | | | |
| | | sphere | infinity | 0.023 | | | | |

(continued)

| Fourth embodiment | | | | | | | | |
|---|---|---|---|---|---|---|---|---|
| f=0.621mm, FNO=3.8, FOV=130deg, TTL=2.38mm | | | | | | | | |
| Surface number | Surface name | Surface type | Y radius | Thickness | Material | Refractive index | Abbe number | Focal length |
| STO | Aperture | sphere | infinity | -0.013 | | | | |
| 3 | Second lens | asphere | 0.685 | 0.302 | plastic | 1.537 | 55.75 | 1.335 |
| 4 | | asphere | 13.357 | 0.030 | | | | |
| 5 | Third lens | asphere | 0.443 | 0.281 | plastic | 1.546 | 56.11 | 0.477 |
| 6 | | asphere | -0.489 | 0.03 | | | | |
| 7 | Fourth lens | asphere | -0.572 | 0.21 | plastic | 1.667 | 20.38 | -0.811 |
| 8 | | asphere | 11.352 | 0.483 | | | | |
| CG | Protective glass | sphere | infinity | 0.4 | glass | | | |
| | | sphere | infinity | 0.0324 | | | | |
| IMG | Imaging surface | sphere | infinity | 0 | | | | |

Table 6

| Fourth embodiment | | | | | |
|---|---|---|---|---|---|
| Surface number | K | A4 | A6 | A8 | A10 |
| 1 | -1.74789E+01 | 3.84417E+00 | 1.59505E+01 | -8.49361E+02 | 1.12208E+04 |
| 2 | -1.47477E+01 | 3.16140E+01 | -1.77691E+03 | 1.88894E+05 | -1.27065E+07 |
| 3 | -1.82042E+01 | 8.65180E+00 | -6.65354E+02 | 4.42414E+04 | -1.30634E+06 |
| 4 | -9.90000E+01 | -3.99302E+01 | 1.71286E+03 | -1.39347E+05 | 1.00897E+07 |
| 5 | -1.71563E+01 | -1.31927E+01 | 6.99677E+02 | -5.82748E+04 | 2.95774E+06 |
| 6 | -2.18174E+00 | 1.75454E+01 | -7.90310E+02 | 3.68972E+04 | -1.49557E+06 |
| 7 | 1.18712E+00 | 1.67951E+01 | -5.69263E+02 | 1.40790E+04 | -4.52551E+05 |
| 8 | -9.90000E+01 | 6.30581E+00 | -1.06858E+02 | 4.86857E+02 | 4.69074E+03 |
| Surface number | A12 | A14 | A16 | A18 | A20 |
| 1 | -8.17736E+04 | 3.62301E+05 | -9.70657E+05 | 1.44746E+06 | -9.22812E+05 |
| 2 | 5.30738E+08 | -1.38727E+10 | 2.22255E+11 | -1.99945E+12 | 7.76369E+12 |
| 3 | -9.31642E+07 | 1.08153E+10 | -4.40325E+11 | 8.39277E+12 | -6.25591E+13 |
| 4 | -5.35265E+08 | 1.83062E+10 | -3.78152E+11 | 4.29482E+12 | -2.06223E+13 |
| 5 | -9.23431E+07 | 1.64078E+09 | -1.48988E+10 | 4.98966E+10 | 4.51436E+10 |
| 6 | 4.06651E+07 | -6.75242E+08 | 6.56202E+09 | -3.42858E+10 | 7.44553E+10 |
| 7 | 1.16291E+07 | -1.76213E+08 | 1.49124E+09 | -6.57069E+09 | 1.17699E+10 |
| 8 | -7.71656E+04 | 4.66909E+05 | -1.47829E+06 | 2.41047E+06 | -1.58111E+06 |

Referring to FIG. 8, it can be seen from (A) a longitudinal spherical aberration diagram, (B) an astigmatism curve diagram, and (C) a distortion diagram in FIG. 8 that, the spherical aberration value, astigmatism, and distortion of the optical lens 100 are well controlled, so that the optical lens 100 of this embodiment has a good imaging quality. In addition, regarding the wavelengths corresponding to the curves in (A) in Figure 8, (B) in Figure 8, and (C) in Figure 8, please refer to the contents described in (A) in Figure 2, (B) in Figure 2, and (C) in Figure 2 in the first embodiment and will not be described again here.

Fifth embodiment

Referring to FIG. 9, the optical lens 100 sequentially includes a first lens L1, a filter IR, an aperture STO, a second lens L2, a third lens L3, a fourth lens L4, and a protective glass CG.

In the illustrated embodiment, the first lens L1 has negative refractive power, an object side surface S1 and an imaging side surface S2 of the first lens L1 are respectively convex and concave near the optical axis O. The second lens L2 has positive refractive power, an object side surface S3 and an imaging side surface S4 of the second lens L2 are respectively convex and concave near the optical axis O. The third lens L3 has positive refractive power, an object side surface S5 and an imaging side surface S6 of the third lens L3 are both convex near the optical axis O. The fourth lens L4 has negative refractive power, an object side surface S7 and an imaging side surface S8 of the fourth lens L4 are both concave near the optical axis O.

The parameters of the optical lens 100 are given in Table 9 below. The definitions of each parameter can be obtained from the description of the previous embodiments, which will not be repeated here. Correspondingly, Table 10 below shows the high-order coefficients of the aspheric lenses that can be used in the fifth embodiment.

Table 9

| Fifth embodiment | | | | | | | | |
|---|---|---|---|---|---|---|---|---|
| f=0.509mm, FNO=4.4, FOV=145deg, TTL=2.49mm | | | | | | | | |
| Surface number | Surface name | Surface type | Y radius | Thickness | Material | Refractive index | Abbe number | Focal length |
| OBJ | Object side | sphere | infinity | 10 | | | | |
| 1 | First lens | asphere | 22.147 | 0.206 | plastic | 1.538 | 56.01 | -0.424 |
| 2 | | asphere | 0.225 | 0.189 | | | | |
| IR | Filter | sphere | infinity | 0.21 | glass | | | |
| | | sphere | infinity | 0.040 | | | | |
| STO | Aperture | sphere | infinity | -0.002 | | | | |
| 3 | Second lens | asphere | 0.805 | 0.307 | plastic | 1.537 | 55.75 | 1.549 |
| 4 | | asphere | 22.147 | 0.03 | | | | |
| 5 | Third lens | asphere | 0.434 | 0.364 | plastic | 1.546 | 56.11 | 0.440 |
| 6 | | asphere | -0.378 | 0.02 | | | | |
| 7 | Fourth lens | asphere | -0.561 | 0.21 | plastic | 1.667 | 20.38 | -0.799 |
| 8 | | asphere | 12.3515 | 0.467 | | | | |
| CG | Protective glass | sphere | infinity | 0.4 | glass | | | |
| | | sphere | infinity | 0.0324 | | | | |
| IMG | Imaging surface | sphere | infinity | 0 | | | | |

Table 10

| Fifth embodiment | | | | | |
|---|---|---|---|---|---|
| Surface number | K | A4 | A6 | A8 | A10 |
| 1 | -9.90000E+01 | 2.40491E+00 | -1.60847E+01 | 1.22668E+02 | -1.10491E+03 |
| 2 | -6.77460E-01 | 6.69123E+00 | -5.44677E+02 | 6.59480E+04 | -3.84507E+06 |
| 3 | -2.06440E+01 | 4.26296E+00 | -5.75675E+02 | 7.68545E+04 | -8.62833E+06 |
| 4 | -9.90000E+01 | -3.48665E+01 | -1.57772E+02 | 1.17165E+05 | -9.49098E+06 |
| 5 | -1.90393E+01 | -1.15524E+01 | 1.95103E+02 | -1.43457E+04 | 9.75687E+05 |
| 6 | -1.73645E-01 | 1.04096E+01 | -1.23294E+02 | -6.82525E+03 | 2.64023E+05 |

(continued)

| Fifth embodiment | | | | | |
|---|---|---|---|---|---|
| Surface number | K | A4 | A6 | A8 | A10 |
| 7 | 4.44482E-01 | 1.14705E+01 | -1.85373E+02 | -3.70288E+03 | 1.39700E+05 |
| 8 | 3.64810E+01 | 3.64249E+00 | 6.18094E+00 | -1.00456E+03 | 1.39994E+04 |
| Surface number | A12 | A14 | A16 | A18 | A20 |
| 1 | 7.05157E+03 | -2.76567E+04 | 6.45836E+04 | -8.30923E+04 | 4.55605E+04 |
| 2 | 1.44159E+08 | -3.55618E+09 | 5.46491E+10 | -4.66219E+11 | 1.66598E+12 |
| 3 | 5.79256E+08 | -2.18914E+10 | 4.68621E+11 | -5.38027E+12 | 2.58922E+13 |
| 4 | 4.22583E+08 | -1.15904E+10 | 1.93862E+11 | -1.81336E+12 | 7.28208E+12 |
| 5 | -4.06970E+07 | 9.94723E+08 | -1.43144E+10 | 1.11565E+11 | -3.57982E+11 |
| 6 | -3.68871E+06 | 1.88217E+07 | 5.63533E+07 | -9.70002E+08 | 2.86564E+09 |
| 7 | -1.14003E+06 | -8.52194E+06 | 2.02525E+08 | -1.23619E+09 | 2.54853E+09 |
| 8 | -8.38195E+04 | 1.55618E+05 | 7.26812E+05 | -4.13603E+06 | 5.92040E+06 |

Referring to FIG. 10, it can be seen from (A) a longitudinal spherical aberration diagram, (B) an astigmatism curve diagram, and (C) a distortion diagram in FIG. 10 that, the spherical aberration value, astigmatism, and distortion of the optical lens 100 are well controlled, so that the optical lens 100 of this embodiment has a good imaging quality. In addition, regarding the wavelengths corresponding to the curves in (A) in Figure 10, (B) in Figure 10, and (C) in Figure 10, please refer to the contents described in (A) in Figure 2, (B) in Figure 2, and (C) in Figure 2 in the first embodiment and will not be described again here.

Referring to Table 11, Table 11 is a summary of the ratios of each relational expression in the first to fifth embodiments of the present disclosure.

Table 11

| Relational expression/embodiment | First embodiment | Second embodiment | Third embodiment | Fourth embodiment | Fifth embodiment |
|---|---|---|---|---|---|
| f1/f | -0.852 | -0.969 | -0.894 | -1.185 | -0.832 |
| f2/f | 1.693 | 2.523 | 2.149 | 1.970 | 3.043 |
| f3/f | 0.724 | 0.742 | 0.768 | 0.788 | 0.863 |
| f4/f | -1.117 | -1.376 | -1.306 | -1.347 | -1.570 |
| R11/f | -1.200 | -1.759 | -1.398 | -0.753 | 43.511 |
| R12/f | 0.807 | 0.789 | 0.787 | 4.738 | 0.442 |
| R21/f | 0.958 | 1.221 | 1.103 | 1.011 | 1.582 |
| R22/f | -14.815 | 10.392 | 21.509 | 20.598 | 43.511 |
| R31/f | 0.781 | 0.765 | 0.713 | 0.742 | 0.852 |
| R32/f | -0.604 | -0.645 | -0.788 | -0.831 | -0.742 |
| R41/f | -0.609 | -0.674 | -0.921 | -0.948 | -1.101 |
| R42/f | -4.010 | -3.074 | 18.279 | 19.090 | 24.266 |
| FOV (unit: deg) | 132.100 | 139.500 | 130.000 | 130.000 | 145.000 |
| TTL/ImgH | 2.189 | 2.000 | 2.315 | 1.946 | 2.422 |
| TTL/f | 3.333 | 4.085 | 3.833 | 3.091 | 4.892 |
| ImgH/f | 1.523 | 2.043 | 1.655 | 1.589 | 2.020 |
| R11/R12 | -1.487 | -2.230 | -1.776 | -0.159 | 98.443 |

(continued)

| Relational expression/embodiment | First embodiment | Second embodiment | Third embodiment | Fourth embodiment | Fifth embodiment |
|---|---|---|---|---|---|
| R22/R21 | -15.461 | 8.509 | 19.492 | 20.384 | 27.502 |
| R31/R32 | -1.293 | -1.186 | -0.905 | -0.893 | -1.147 |
| R42/R41 | 6.588 | 4.563 | -19.849 | -20.128 | -22.034 |
| CT3/CT4 | 1.736 | 1.514 | 1.340 | 1.220 | 1.734 |
| ET1/CT1 | 1.409 | 1.302 | 1.556 | 1.311 | 1.488 |
| ET4/CT4 | 1.679 | 1.717 | 1.594 | 1.401 | 1.728 |
| (\|SAG11\|+\|SAG12\|)/CT1 | 0.717 | 1.210 | 1.147 | 0.620 | 1.103 |
| (\|SAG21\|+\|SAG22\|)/CT2 | 0.238 | 0.214 | 0.287 | 0.192 | 0.176 |
| (\|SAG31\|+\|SAG32\|)/CT3 | 0.484 | 0.732 | 0.344 | 0.219 | 0.455 |
| (\|SAG41\|+\|SAG42\|)/CT4 | 0.814 | 0.787 | 0.594 | 0.401 | 0.728 |
| f4/\|SAG41\| | -4.811 | -5.121 | -8.575 | -11.399 | -7.739 |
| f4/\|SAG42\| | -53.456 | -110.326 | -26.934 | -111.422 | -16.096 |
| FOV/FNO (unit: deg) | 30.023 | 36.711 | 34.211 | 31.707 | 32.955 |
| TTL/BL | 2.420 | 2.712 | 2.599 | 2.620 | 2.770 |
| TD/ΣAT | 2.834 | 2.889 | 2.645 | 3.034 | 2.893 |
| AT12/AT34 | 11.093 | 12.887 | 13.463 | 6.459 | 21.820 |
| SD11/SD42 | 1.046 | 1.081 | 1.173 | 1.113 | 1.281 |
| SD22/SD31 | 0.921 | 0.921 | 0.925 | 0.840 | 0.889 |
| SD11/ImgH | 0.535 | 0.467 | 0.545 | 0.522 | 0.545 |

Referring to FIG. 11, the present disclosure also discloses an image module 200. The image module 200 includes an imaging sensor 201 and the optical lens 10 as described in any of the above embodiments. The imaging sensor 201 is arranged on the imaging side of the optical lens 10. The optical lens 100 is used to receive the light signal of the object being photographed and project it onto the imaging sensor 201, which converts the light signal corresponding to the object into an image signal, this will not be elaborated here. It can be understood that the image module 200 with the above optical lens 100 has all the technical effects of the optical lens 100 and can achieve wide-angle imaging on the basis of miniaturized design. Since the technical effects have been described in detail in the embodiments of the optical lens 100, they will not be repeated here.

Referring to FIG. 12, the present disclosure also discloses an endoscope 300. The endoscope 300 includes a tube body 301 and the above-mentioned image module 200, the image module 200 is arranged in the tube body 301. It can be understood that the endoscope 300 may include an industrial endoscope or a medical endoscope.

The optical lens, image module and endoscope disclosed in the embodiments of the present invention have been introduced in detail above. Specific examples are used in this application to illustrate the principles and implementation methods of the present invention. The description of the above embodiments is only used to help understand the optical lens, image module and endoscope of the invention and its core idea; at the same time, for those of ordinary skill in the field, there will be changes in the specific implementation and application scope based on the ideas of the present invention. In summary, the content of this description should not be understood as a limitation of the present invention.

## Claims

**1.** An optical lens (100), from an object side to an imaging side along an optical axis (O), sequentially comprising:

a first lens (L1) having negative refractive power, an imaging side surface (S2) of the first lens (L1) being concave near the optical axis (O);
a filter (IR);

a second lens (L2) having positive power, an object side surface (S3) of the second lens (L2) being convex near the optical axis (O);
a third lens (L3) having positive refractive power, an object side surface (S5) of the third lens (L3) being convex near the optical axis (O);
a fourth lens (L4) having negative refractive power, an object side surface (S7) of the fourth lens (L4) being concave near the optical axis (O); and
an image plane (IMG);
the optical lens (100) satisfying following conditional expressions:

$$110deg<FOV<155deg, \text{ and } 1.7<TTL/ImgH<2.7;$$

wherein, FOV is the maximum field of view angle of the optical lens (100), TTL is a distance from an object side surface (S1) of the first lens (L1) to the image plane (IMG) of the optical lens (100) along the optical axis (O), and ImgH is half of an image height corresponding to the maximum field of view of the optical lens (100).

**2.** The optical lens (100) of claim 1, wherein the optical lens (100) further satisfies at least one of following conditional expressions:

$$-1.4<f1/f<-0.6,$$

$$1.2<f2/f<4,$$

$$0.5<f3/f<1.1,$$

and

$$-2<f4/f<-0.8,$$

wherein, f1 is a focal length of the first lens (L1), f is a focal length of the optical lens (100), f2 is a focal length of the second lens (L2), f3 is a focal length of the third lens (L3), and f4 is a focal length of the fourth lens (L4).

**3.** The optical lens (100) of claim 1, wherein the optical lens (100) further satisfies at least one of following conditional expressions:

$$0.7<|R11|/f,$$

$$0.3<R12/f,$$

$$0.6<R21/f<2,$$

$$9<|R22|/f,$$

$$0.5<R31/f<1.0,$$

$$-1.1<R32/<-0.4,$$

$$-1.3<R41/f<-0.4,$$

and

$$3<|R42|/f,$$

wherein, R11 is a radius of curvature of an object side surface (S1) of the first lens (L1) at the optical axis (O), f is a focal length of the optical lens (100), R12 is a radius of curvature of the imaging side surface (S2) of the first lens (L1) at the optical axis (O), R21 is a radius of curvature of the object side surface (S3) of the second lens (L2) at the optical axis (O), R22 is a radius of curvature of an imaging side surface (S4) of the second lens (L2) at the optical axis (O), R31 is a radius of curvature of the object side surface (S5) of the third lens (L3) at the optical axis (O), R32 is a radius of curvature of an imaging side surface (S6) of the third lens (L3) at the optical axis (O), R41 is a curvature radius of the object side surface (S7) of the fourth lens (L4) at the optical axis (O), and R42 is a curvature radius of an imaging side surface (S8) of the fourth lens (L4) at the optical axis (O).

**4.** The optical lens (100) of claim 1, wherein the optical lens (100) further satisfies at least one of following conditional expressions:

$$2.5<TTL/f<5.5,$$

and

$$1.2<ImgH/f<2.3,$$

wherein, f is a focal length of the optical lens (100).

**5.** The optical lens (100) of claim 1, wherein the optical lens (100) further satisfies at least one of following conditional expressions:

$$2.2<TTL/BL<3,$$

and

$$2.5<TD/\Sigma AT<3.5,$$

wherein, BL is a distance from an imaging side surface (S8) of the fourth lens (L4) to the image plane (IMG) of the optical lens (100) along the optical axis (O), TD is a distance from an object side surface (S1) of the first lens (L1) to the imaging side surface (S8) of the fourth lens (L4) at the optical axis (O), and $\Sigma AT$ is a sum of distances from the imaging side surface (S2) of the first lens (L1) to an object side surface of the filter (IR), from an imaging side surface of the filter (IR) to the object side surface (S3) of the second lens (L2), from an imaging side surface (S4) of the second lens (L2) to the object side surface (S5) of the third lens (L3), and from an imaging side surface (S6) of the third lens (L3) to the object side surface (S7) of the fourth lens (L4) at the optical axis (O).

**6.** The optical lens (100) of claim 1, wherein the optical lens (100) further satisfies at least one of following conditional expressions:

$$3<FNO<5,$$

and

$$26deg<FOV/FNO<40deg,$$

wherein, FNO is an aperture number of the optical lens (100).

**7.** The optical lens (100) of claim 1, wherein the optical lens (100) further satisfies at least one of following conditional expressions:

$$0.9<CT3/CT4<2.1,$$

$$1<ET1/CT1<1.9,$$

$$1<ET4/CT4<2.1,$$

and

$$6<AT12/AT34<22,$$

wherein, CT3 is a thickness of the third lens (L3) at the optical axis (O), CT4 is a thickness of the fourth lens (L4) at the optical axis (O), ET1 is a distance from the maximum effective semi-aperture of an object side surface (S1) of the first lens (L1) to the maximum effective semi-aperture of the imaging side surface (S2) of the first lens (L1), ET4 is a distance from the maximum effective semi-aperture of the object side surface (S7) of the fourth lens (L4) to the maximum effective semi-aperture of an imaging side surface (S8) of the fourth lens (L4), AT12 is a sum of distances from the imaging side surface (S2) of the first lens (L1) to an object side surface of the filter (IR) and from an imaging side surface of the filter (IR) to the object side surface (S3) of the second lens (L2) at the optical axis (O), and AT34 is a distance from an imaging side surface (S6) of the third lens (L3) to the object side surface (S7) of the fourth lens (L4) at the optical axis (O).

**8.** The optical lens (100) of claim 1, wherein the optical lens (100) further satisfies at least one of following conditional expressions:

$$1<SD11/SD42<1.3,$$

$$0.8<SD22/SD31<1,$$

and

$$0.4<SD11/ImgH<0.6,$$

wherein, SD11 is the maximum effective semi-aperture of an object side surface (S1) of the first lens (L1), SD42 is the maximum effective semi-aperture of an imaging side surface (S8) of the fourth lens (L4), SD22 is the maximum effective semi-aperture of an imaging side surface (S4) of the second lens (L2), and SD31 is the maximum effective semi-aperture of the object side surface (S5) of the third lens (L3).

**9.** The optical lens (100) of claim 1, wherein the optical lens (100) further satisfies at least one of following conditional expressions:

$$0.5<(|SAG11|+|SAG12|)/CT1<1.5,$$

$$0.1<(|SAG21|+|SAG22|)/CT2<0.4,$$

$$0.1<(|SAG31|+|SAG32|)/CT3<0.9,$$

and

$$0.3<(|SAG41|+|SAG42|)/CT4<1,$$

wherein, CT1 is a thickness of the first lens (L1) at the optical axis (O), SAG11 is a distance from the maximum effective semi-aperture of an object side surface (S1) of the first lens (L1) to an intersection point of the object side surface (S1) of the first lens (L1) and the optical axis (O) in the direction of the optical axis (O), SAG12 is a distance from the maximum effective semi-aperture of the imaging side surface (S2) of the first lens (L1) to an intersection point of the imaging side surface (S2) of the first lens (L1) and the optical axis (O) in the direction of the optical axis (O), CT2 is a thickness of the second lens (L2) at the optical axis (O), SAG21 is a distance from the maximum effective semi-aperture of the object side surface (S3) of the second lens (L2) to an intersection point of the object side surface (S3) of the second lens (L2) and the optical axis (O) in the direction of the optical axis (O), SAG22 is a distance from the maximum effective semi-aperture of an imaging side surface (S4) of the second lens (L2) to an intersection point of the imaging side surface (S4) of the second lens (L2) and the optical axis (O) in the direction of the optical axis (O), CT3 is a thickness of the third lens (L3) at the optical axis (O), SAG31 is a distance from the maximum effective semi-aperture of the object side surface (S5) of the third lens (L3) to an intersection point of the object side surface (S5) of the third lens (L3) and the optical axis (O) in the direction of the optical axis (O), SAG32 is a distance from the maximum effective semi-aperture of an imaging side surface (S6) of the third lens (L3) to an intersection point of the imaging side surface (S6) of the third lens (L3) and the optical axis (O) in the direction of the optical axis (O), CT4 is a thickness of the fourth lens (L4) at the optical axis (O), SAG41 is a distance from the maximum effective semi-aperture of the object side surface (S7) of the fourth lens (L4) to an intersection point of the object side surface (S7) of the fourth lens (L4) and the optical axis (O) in the direction of the optical axis (O), and SAG42 is a distance from the maximum effective semi-aperture of an imaging side surface (S8) of the fourth lens (L4) to an intersection point of the imaging side surface (S8) of the fourth lens (L4) and the optical axis (O) in the direction of the optical axis (O).

**10.** The optical lens (100) of claim 1, wherein the optical lens (100) further satisfies at least one of following conditional expressions:

$$-14<f4/|SAGY41|<-3,$$

and

$$f4/|SAGY42|<-10,$$

wherein, SAG41 is a distance from the maximum effective semi-aperture of the object side surface (S7) of the fourth lens (L4) to an intersection point of the object side surface (S7) of the fourth lens (L4) and the optical axis (O) in the direction of the optical axis (O), SAG42 is a distance from the maximum effective semi-aperture of an imaging side surface (S8) of the fourth lens (L4) to an intersection point of the imaging side surface (S8) of the fourth lens (L4) and the optical axis (O) in the direction of the optical axis (O), and f4 is a focal length of the fourth lens (L4).

**11.** The optical lens (100) of claim 1, wherein the optical lens (100) further satisfies at least one following conditional expressions:

$$2.3<R11/R12<100,$$

$$7<|R22/R21|<30,$$

$$-1.7<R31/R32<-0.6,$$

and

$$4<|R42/R41|,$$

wherein, R11 is a radius of curvature of an object side surface (S1) of the first lens (L1) at the optical axis (O), R12 is a radius of curvature of the imaging side surface (S2) of the first lens (L1) at the optical axis (O), R21 is a radius of curvature of the object side surface (S3) of the second lens (L2) at the optical axis (O), R22 is a radius of curvature of an imaging side surface (S4) of the second lens (L2) at the optical axis (O), R31 is a radius of curvature of the object side surface (S5) of the third lens (L3) at the optical axis (O), R32 is a radius of curvature of an imaging side surface (S6) of the third lens (L3) at the optical axis (O), R41 is a curvature radius of the object side surface (S7) of the fourth lens (L4) at the optical axis (O), and R42 is a curvature radius of an imaging side surface (S8) of the fourth lens (L4) at the optical axis (O).

**12.** The optical lens (100) of claim 1, wherein the optical lens (100) further comprises an aperture, and the aperture is arranged between the filter (IR) and the object side surface (S3) of the second lens (L2).

**13.** The optical lens (100) of claim 1, wherein the optical lens (100) further comprises a protective glass, and the protective glass is arranged between an imaging side surface (S8) of the fourth lens (L4) and the image plane (IMG) of the optical lens.

**14.** An image module (200) comprising an imaging sensor (201) and the optical lens (100) of any one of claims 1 to 13, the imaging sensor (201) is arranged on the imaging side of the optical lens (100).

**15.** An endoscope (300) comprising a tube body (301) and the image module (200) of claim 14, the image module (200) being arranged in the tube body (301).

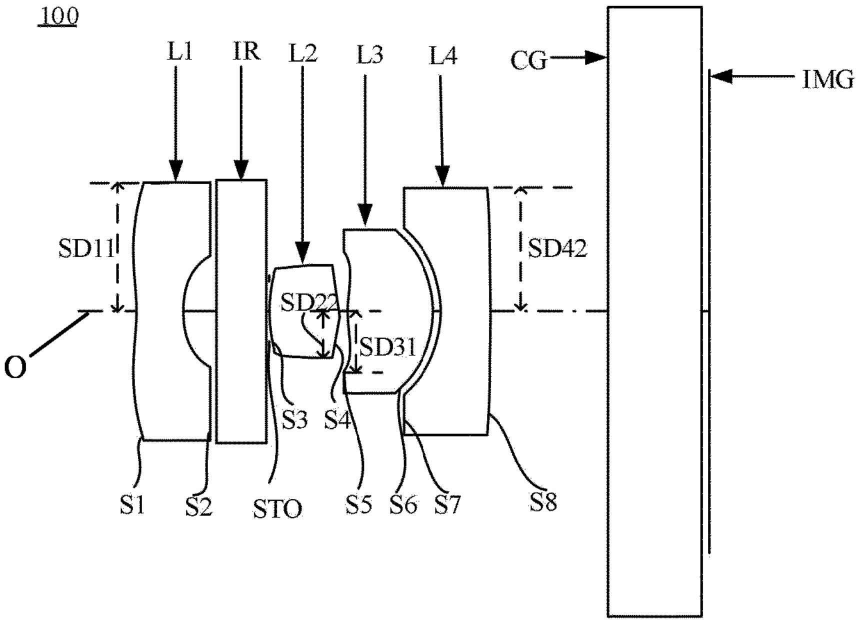
100
L1
IR
L2
L3
L4
CG
IMG
SD11
SD42
SD22
SD31
O
S3
S4
S1
S2
STO
S5
S6
S7
S8

FIG.1A

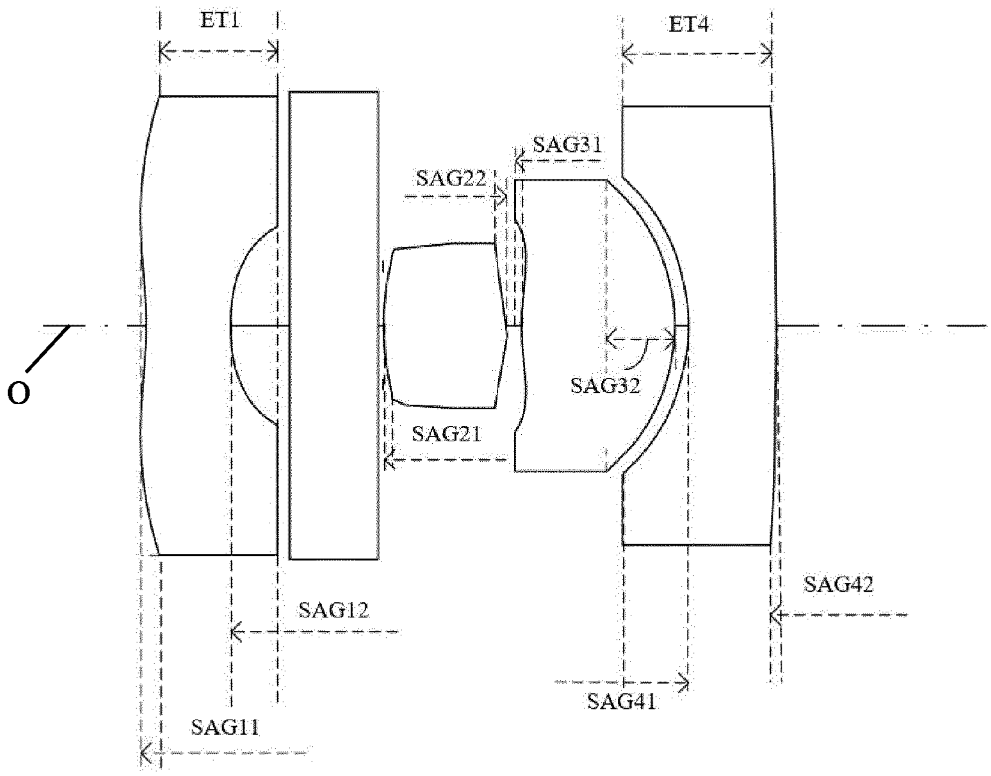
ET1
ET4
SAG31
SAG22
O
SAG32
SAG21
SAG42
SAG12
SAG41
SAG11

FIG.1B

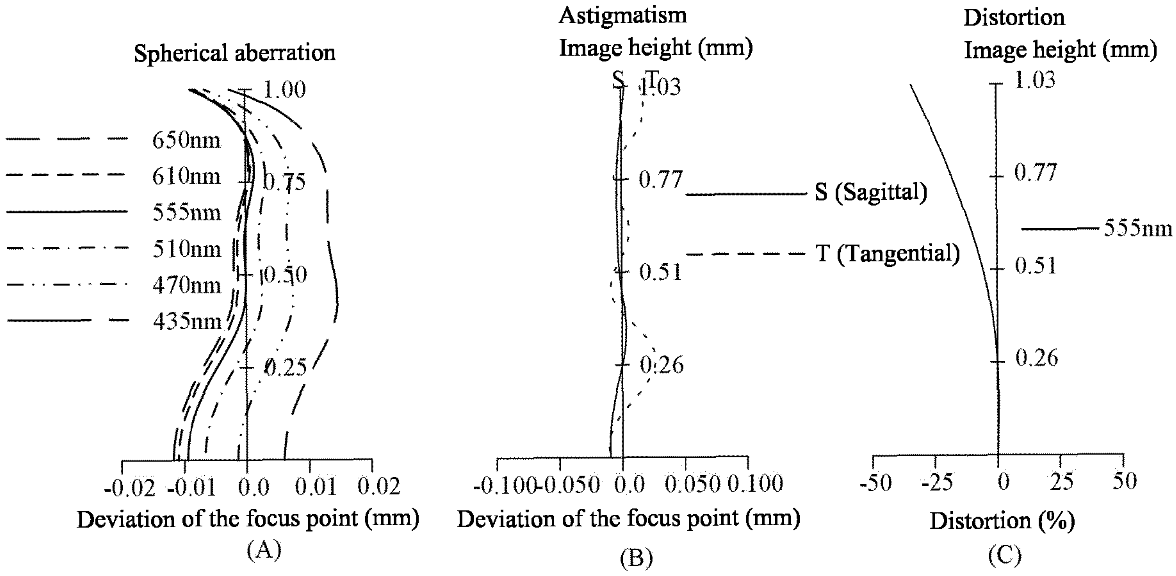
Spherical aberration
1.00
0.75
0.50
0.25
650nm
610nm
555nm
510nm
470nm
435nm
-0.02 -0.01 0.0 0.01 0.02
Deviation of the focus point (mm)
(A)
Astigmatism
Image height (mm)
S T
1.03
0.77
0.51
0.26
S (Sagittal)
T (Tangential)
-0.100 -0.050 0.0 0.050 0.100
Deviation of the focus point (mm)
(B)
Distortion
Image height (mm)
1.03
0.77
0.51
0.26
555nm
-50 -25 0 25 50
Distortion (%)
(C)

FIG.2

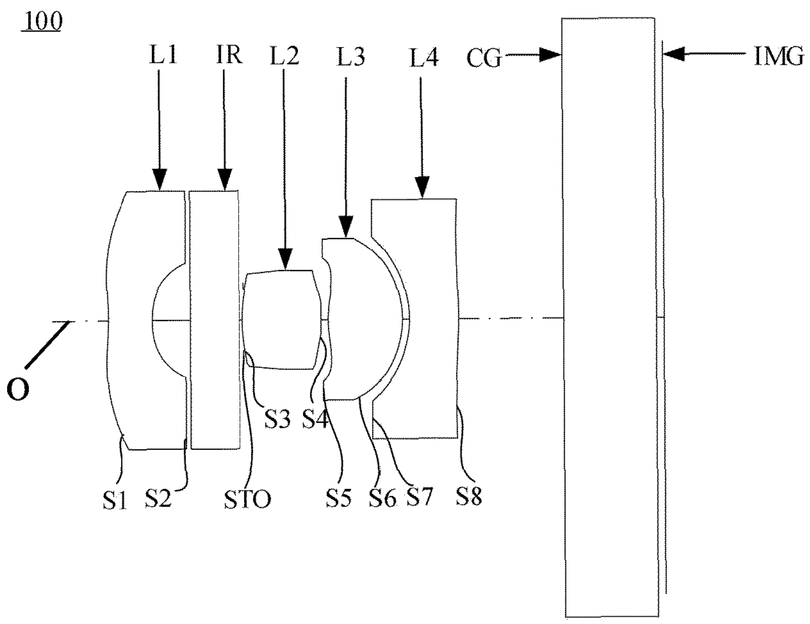
100
L1
IR
L2
L3
L4
CG
IMG
O
S1 S2
STO
S3 S4
S5 S6 S7 S8

FIG.3

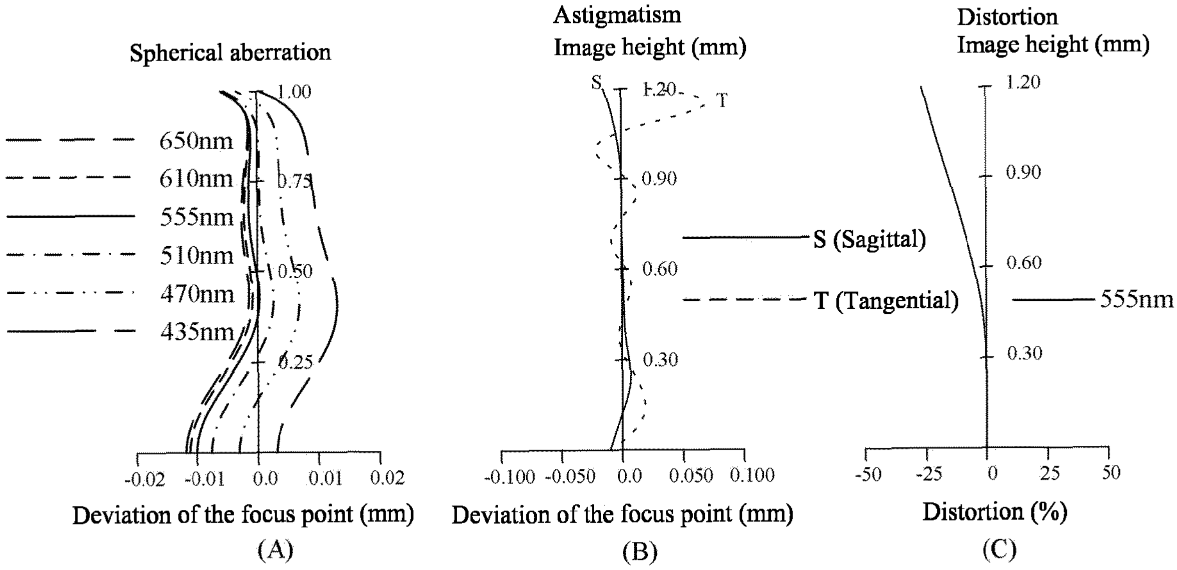
Spherical aberration
650nm
610nm
555nm
510nm
470nm
435nm
1.00
0.75
0.50
0.25
-0.02
-0.01
0.0
0.01
0.02
Deviation of the focus point (mm)
(A)
Astigmatism
Image height (mm)
S
T
1.20
0.90
0.60
0.30
S (Sagittal)
T (Tangential)
-0.100
-0.050
0.0
0.050
0.100
Deviation of the focus point (mm)
(B)
Distortion
Image height (mm)
1.20
0.90
0.60
0.30
555nm
-50
-25
0
25
50
Distortion (%)
(C)

FIG.4

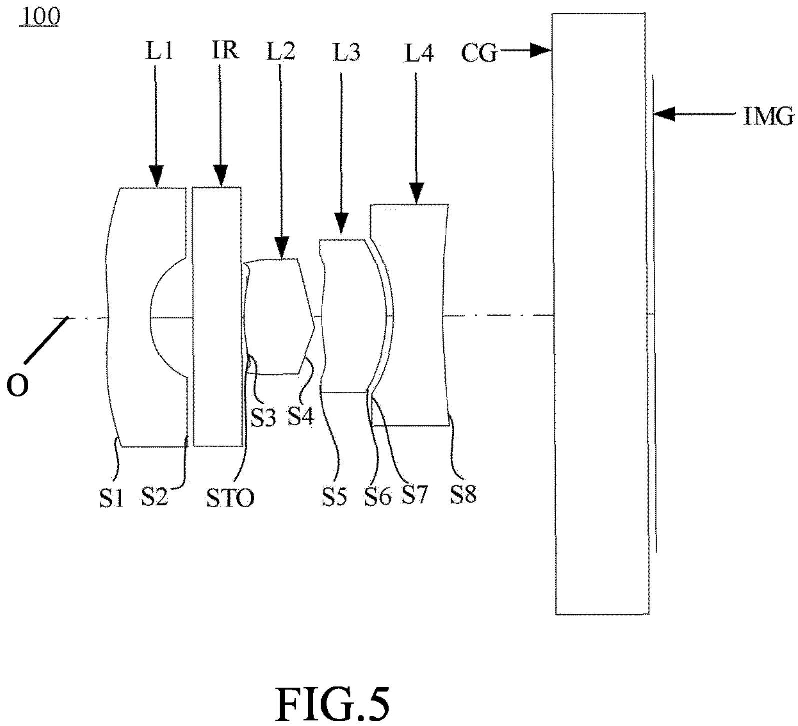
100
L1
IR
L2
L3
L4
CG
IMG
O
S1
S2
STO
S3
S4
S5
S6
S7
S8

FIG.5

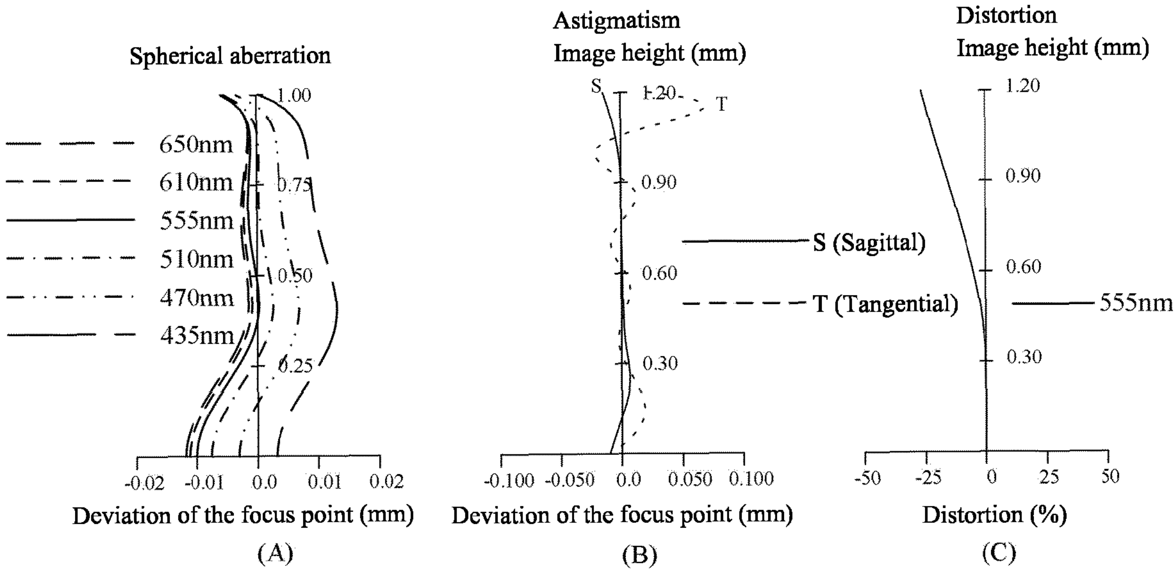
Spherical aberration
650nm
610nm
555nm
510nm
470nm
435nm
1.00
0.75
0.50
0.25
-0.02
-0.01
0.0
0.01
0.02
Deviation of the focus point (mm)
(A)
Astigmatism
Image height (mm)
S
T
1.20
0.90
0.60
0.30
-0.100
-0.050
0.0
0.050
0.100
Deviation of the focus point (mm)
S (Sagittal)
T (Tangential)
(B)
Distortion
Image height (mm)
1.20
0.90
0.60
0.30
555nm
-50
-25
0
25
50
Distortion (%)
(C)

FIG.6

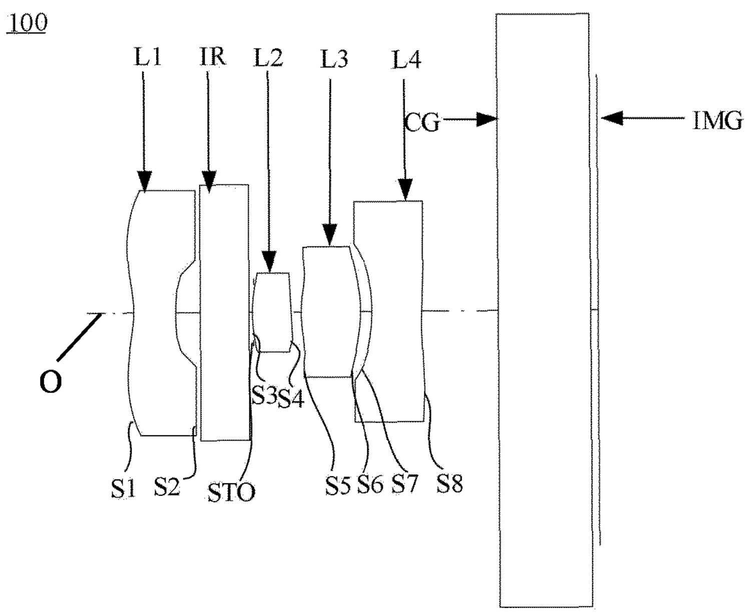
100
L1
IR
L2
L3
L4
CG
IMG
O
S1
S2
STO
S3
S4
S5
S6
S7
S8

FIG.7

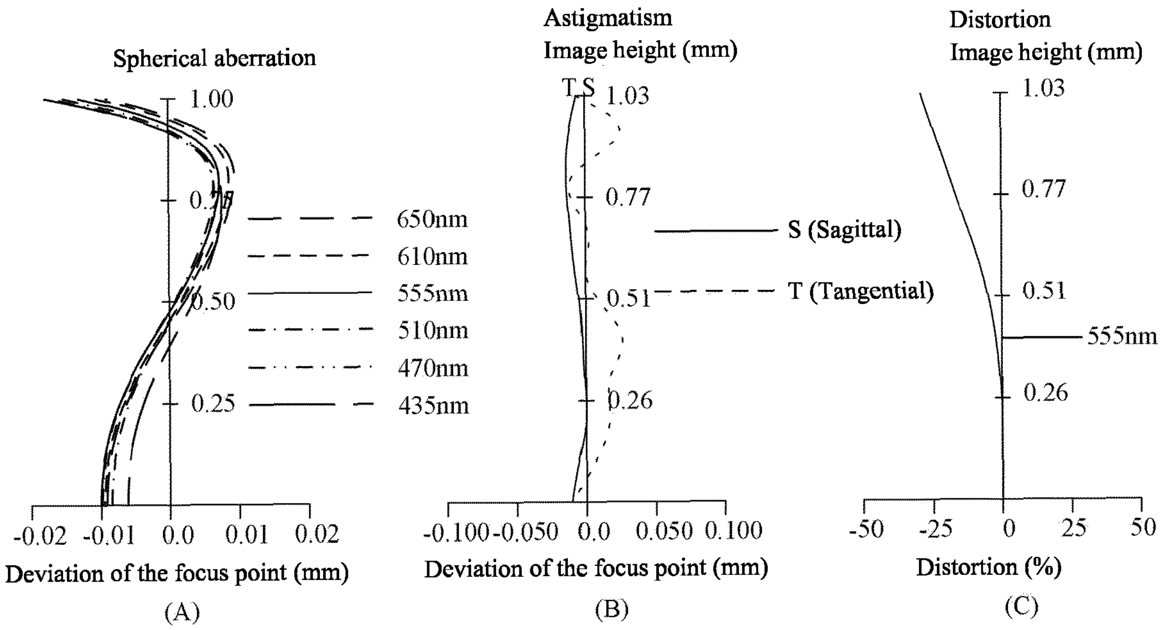
Spherical aberration
1.00
0.75
0.50
0.25
650nm
610nm
555nm
510nm
470nm
435nm
-0.02 -0.01 0.0 0.01 0.02
Deviation of the focus point (mm)
(A)
Astigmatism
Image height (mm)
T S
1.03
0.77
0.51
0.26
S (Sagittal)
T (Tangential)
-0.100 -0.050 0.0 0.050 0.100
Deviation of the focus point (mm)
(B)
Distortion
Image height (mm)
1.03
0.77
0.51
0.26
555nm
-50 -25 0 25 50
Distortion (%)
(C)

FIG.8

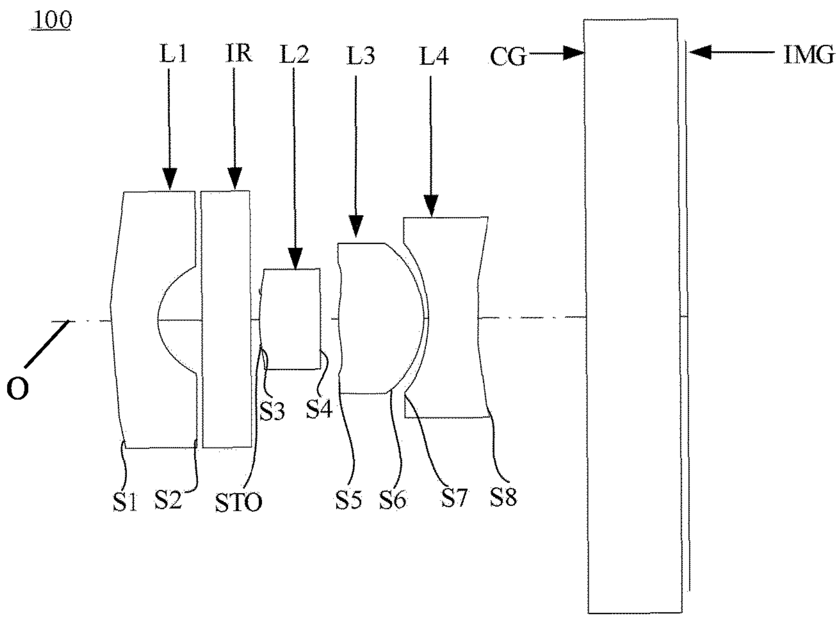
100
L1 IR L2 L3 L4 CG IMG
O
S1 S2 STO S3 S4 S5 S6 S7 S8

FIG.9

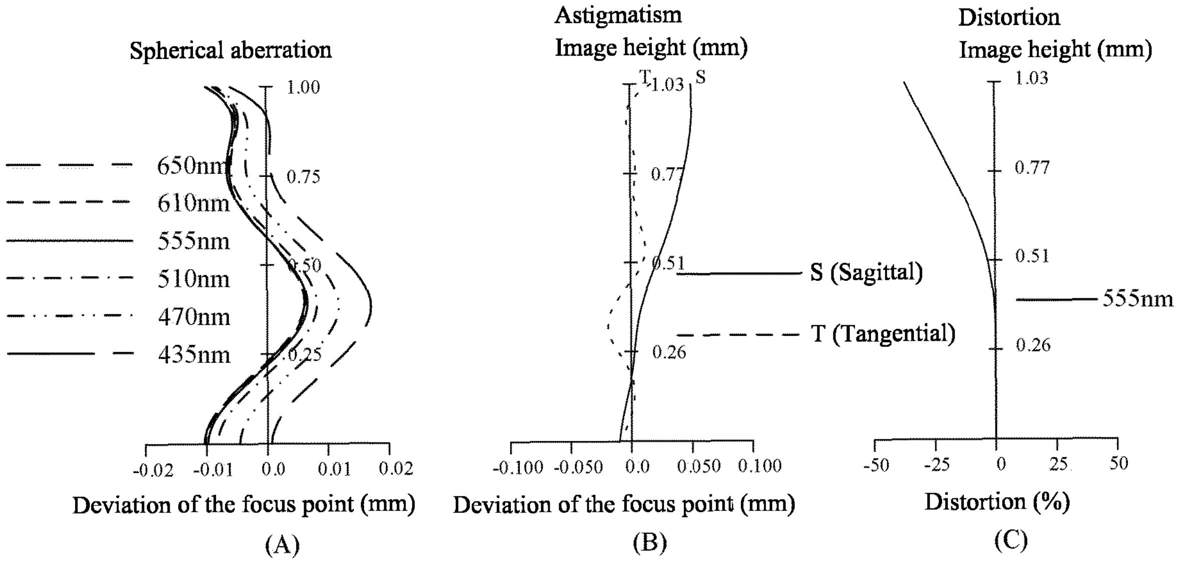
Spherical aberration
650nm
610nm
555nm
510nm
470nm
435nm
1.00
0.75
0.50
0.25
-0.02 -0.01 0.0 0.01 0.02
Deviation of the focus point (mm)
(A)
Astigmatism
Image height (mm)
T 1.03 S
0.77
0.51
0.26
S (Sagittal)
T (Tangential)
-0.100 -0.050 0.0 0.050 0.100
Deviation of the focus point (mm)
(B)
Distortion
Image height (mm)
1.03
0.77
0.51
0.26
555nm
-50 -25 0 25 50
Distortion (%)
(C)

FIG.10

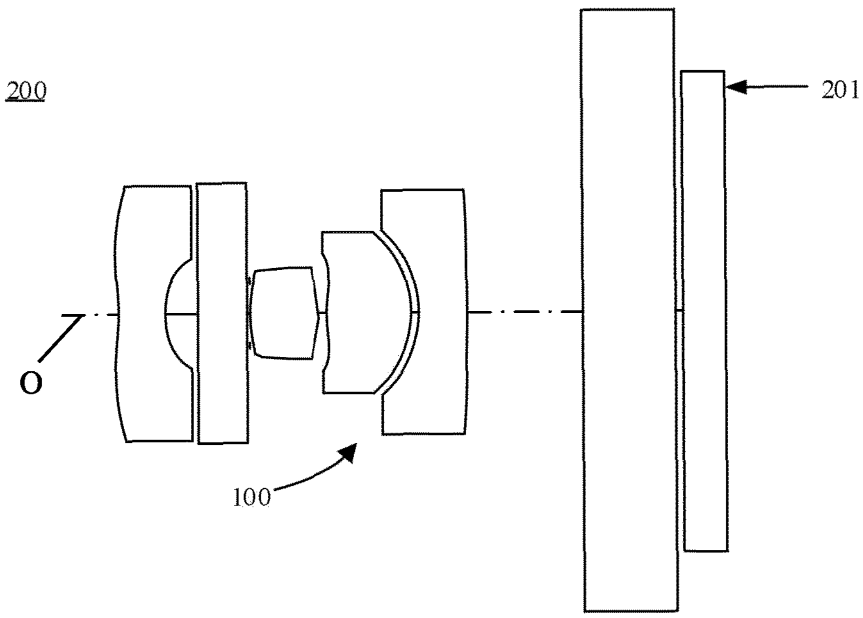
200
201
O
100

FIG.11

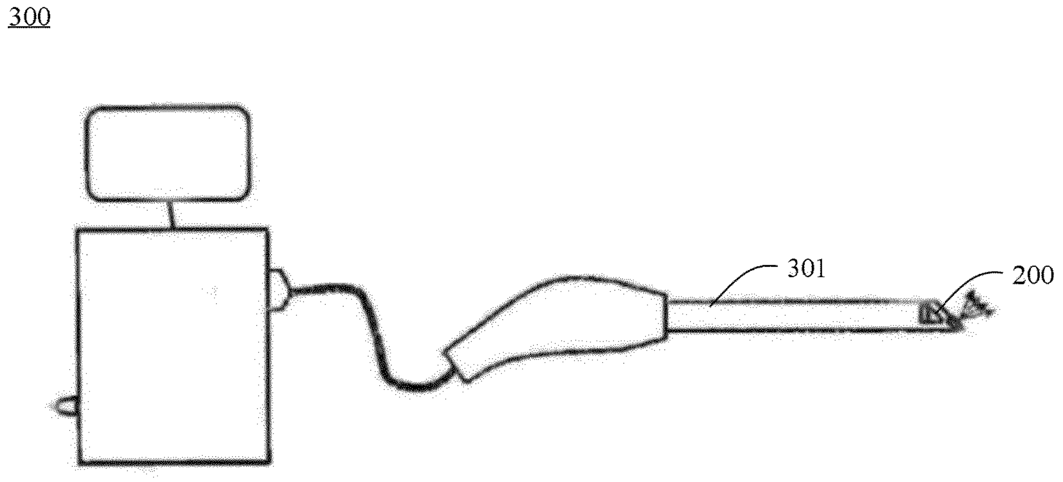


FIG.12